(19) **Europäisches Patentamt**
**European Patent Office**
**Office européen des brevets**

(11) **EP 1 471 957 B1**

(12) **EUROPEAN PATENT SPECIFICATION**

(45) Date of publication and mention
of the grant of the patent:
**12.03.2008 Bulletin 2008/11**

(51) Int Cl.:
**A61M 1/16** (2006.01)   **A61M 1/34** (2006.01)

(21) Application number: **02783349.0**

(22) Date of filing: **31.10.2002**

(86) International application number:
**PCT/IB2002/004537**

(87) International publication number:
**WO 2003/066135 (14.08.2003 Gazette 2003/33)**

(54) **Apparatus and its software for determining blood-flow during dialysis**

Gerät und die dazu gehörende Software zur Bestimmung der Blutströmung bei Dialyse

Appareil et son programme pour déterminer le débit du sang pendant une dialyse

(84) Designated Contracting States:
**AT BE BG CH CY CZ DE DK EE ES FI FR GB GR
IE IT LI LU MC NL PT SE SK TR**

(30) Priority: **08.02.2002 SE 0200370**

(43) Date of publication of application:
**03.11.2004 Bulletin 2004/45**

(60) Divisional application:
**08001550.6**

(73) Proprietor: **Gambro Lundia AB
22 643 Lund (SE)**

(72) Inventors:
• **BENE, Bernard
F-69540 Irigny (FR)**
• **GOUX, Nicolas
F-69290 Craponne (FR)**

• **HANSSON, Per
216 16 Limhamn (SE)**
• **HERTZ, Thomas
S-227 32 Lund (SE)**
• **JANSSON, Olof
S-235 38 Vellinge (SE)**
• **PERSSON, Roland
S-216 11 Limhamn (SE)**
• **STERNBY, Jan
S-226 52 Lund (SE)**
• **ASBRINK, Perry
S-215 81 Malmö (SE)**

(74) Representative: **Villanova, Massimo et al
Gambro Intellectual Property Department,
P.O. Box 98
41037 Mirandola (MO) (IT)**

(56) References cited:
**EP-A- 0 658 352          EP-A- 0 928 614
WO-A-00/24440**

**EP 1 471 957 B1**

**Description**

TECHNICAL FIELD

The present invention relates to an apparatus and a software for determining fluid flow rate in a patient's blood access. More particularly, the invention relates to the calculation of the fluid flow rate in the blood access based on conductivity measurements of the post dialyzer or other blood treatment unit effluent fluid.

BACKGROUND ART

There are several types of treatments in which blood is taken out in an extracorporeal blood circuit. Such treatments involve, for example, hemodialysis, hemofiltration, hemodiafiltration, plasmapheresis, blood component separation, blood oxygenation, etc. Normally, blood is removed from a blood vessel at a blood access and returned to the same blood vessel.

In hemodialysis and similar treatments, a blood access commonly surgically created in the nature of a arterio-venous shunt, commonly referred to as a fistula. Blood needles are inserted in the fistula. Blood is taken out from the fistula via a needle at an upstream position and blood is returned to the fistula via needle at a downstream position.

The arterio-venous shunt or fistula is blood access having capability of providing a high blood flow and being operative during several years and even tens of years. It is produced by operatively connecting, for example, the radial artery to the cephalic vein at the level of the forearm. The venous limb of the fistula thickens during the course of several months, permitting repeated insertion of dialysis needles.

An alternative blood access to the fistula is the arterio-venous graft, in which a connection is generated from, for example, the radial artery at the wrist to the basilic vein. The connection is made with a tube graft made from e.g. autogenous saphenous vein or from polytetrafluoroethylene (PTFE, Teflon). The needles are inserted in the graft.

A further example of a blood access is a silicon, dual-lumen catheter surgically implanted into one of the large veins. Further type of blood access find use in specific situations, like a no-needle arterio-venous graft consisting of a T-tube linked to a standard PTFE graft. The T-tube is implanted in the skin. Vascular access is obtained either by unscrewing a plastic plug or by puncturing a septum of said T-tube with a needle. Other methods and devices are also known.

During the above blood treatment therapies, hemodialysis for instance, it is desirable to obtain a constant blood flow rate of 150 - 500 ml/min or even higher, and the access site must be prepared for delivering such flow rates. The blood flow in an AV fistula is often 800 ml/min or larger, permitting delivery of a blood flow rate in the desired range.

In the absence of a sufficient forward blood flow, the extracorporeal circuit blood pump will take up some of the already treated blood entering the fistula via the venous needle, so called access or fistula recirculation, leading to poor treatment results and progressive reduction of treatment efficiency.

A common cause of poor flow with AV fistulas is partial obstruction of the venous limb due to fibrosis secondary to multiple venipunctures. Moreover, stenosis causes a reduction of access flow.

It has been found that access flow rate often exhibit a long plateau time period with sufficient access flow, followed by a short period of a few weeks with markedly reduced access flow leading to recirculation and ultimately access failure. By constantly monitoring the evolution of the access flow during consecutive treatment sessions, it is possible to detect imminent access flow problems. Proper detection of access flow reduction may help in carrying out a maintenance procedure on the access thereby avoiding any access failure.

A non-invasive technique that allows measurement of flow through AV fistulas and grafts is colour Doppler ultrasound. Magnetic Resonance Imaging (MRI) has also been used. However, these techniques require expensive equipment and are not easily used in the dialysis clinic environment.

Several methods have been suggested for monitoring recirculation and access flow. Many of these methods involve injection of a marker substance in blood, and the resultant recirculation is detected. The methods normally involve measurement of a property in the extracorporeal blood circuit. Examples of such methods can be found in US 5,685,989, US 5,595,182, US 5,453,576, US 5,510,716, US 5,510,717, US 5,392,550, etc.

Such methods have the disadvantage that they require the injection of the marker substance and external equipment for the measurements.

More recently, EP 928 614 and WO 00/24440, suggest to measure a post dialyzer concentration of a substance, in particular urea in the effluent fluid before and after a flow reversal, i.e. before the flow reversal the arterial line carries blood from an upstream position of the blood access, and the venous line carries blood towards a downstream position of the blood access, whereas the arterial line carries blood from an downstream position of the blood access, and the venous line carries blood towards a upstream position of the blood access after the flow reversal.

More particularly, EP 0 928 614 shows a system for measuring fistula flow as a function of a first dialysance or urea concentration value corresponding to a first configuration of the blood lines and function of a second dialysance or urea concentration value corresponding to a second configuration of the bloodlines.

A valve for such reversal is shown in i.e. US 5,605,630 and US 5,894,011. A disadvantage in these methods is the requirement for special equipment for measuring the urea concentration. Urea sensors are as such available but they are not standard equipment for most of the dialysis monitors and they have also a considerable maintenance costs.

SUMMARY OF THE INVENTION

On this background, it is the object of the present invention to provide an apparatus for implementing the method of the kind referred to initially, which is less expensive, easier to implement and easier to operate. This object is achieved in accordance with claim 1 by creating a concentration difference between the blood and the dialysis liquid, and measuring the post dialyzer concentration or conductivity before and after a flow reversal during T when the concentration $C_i$ of at least one substance in the liquid at the treatment unit inlet is kept substantially constant. The creation of a difference in concentration for the purpose of measuring the fluid flow in the blood access allows for a significant increase in precision of the measurement.
It is another object of the invention to provide a software comprising instructions executable by a control unit of a blood treatment apparatus, a dialysis apparatus for instance, of the kind referred to above, able to measure blood access flow, less expensive and easier to operate than the known apparatuses. This object is achieved in accordance with claim 17.
By providing means for creating a difference in conductivity between the dialysis fluid and blood and by providing a post treatment unity conductivity cell, the apparatus can determine the blood access flow, with relatively inexpensive modifications to conventional dialysis apparatuses.
According to a preferred embodiment, a first and second concentration or conductivity are measured on the post treatment unit fluid flowing downstream the treatment unit, or so called effluent fluid.
During normal dialysis a blood flow in a first direction is created by operating a blood pump, in which the arterial line carries blood from said upstream position of said blood access, and the venous line carries blood towards said downstream position of said blood access (normal configuration of the lines).
A blood flow in a second direction, in which said arterial line carries blood from said downstream position of said blood access, and said venous line carries blood towards said upstream portion of said blood access (reversed configuration of the lines), may be created by

-   manually connecting the arterial line to the downstream position of the blood access and the venous line to an upstream position of the blood access, or by
-   connecting the arterial line to both the upstream and the downstream position of the blood access and connecting the venous line to both the upstream and the downstream position of the blood access, closing one of the connections between the arterial line with the blood access and opening the other and closing one of the connections between the venous line with the blood access and opening the other, or by
-   providing a valve able to connect the arterial line with the upstream position of the access point and the venous line with the downstream position of the access point in a first position of said valve and able to connect the arterial line with the downstream position of the access point and the venous line with the upstream position of the access point in a second position of said valve.

The calculation of the fluid flow rate in the blood access is carried out by using the formula:

$$Qa = f(Cr, Ci, Cn, Quf, Tr);$$

According to an embodiment the following formula can be used:

$$Qa = (Tr - Quf)*(Cr - Ci)/(Cn - Cr),$$

In which $Qa$ is the fluid flow rate in the blood access, $Tr$ transport rate of substances over the semi permeable membrane of the treatment unit referred to the venous and arterial lines in normal condition, $Quf$ is the ultrafiltration flow rate, $Cr$ is the post treatment unit conductivity after flow reversal, $Ci$ is pre treatment unit conductivity, and $Cn$ is the post treatment unit conductivity before the flow reversal.
For determination of the transport rate $Tr$, the effective ionic dialysance $D$ can be used. The effective ionic dialysance $D$ determined for example as described in EP 658 352. Alternatively, the transport rate can be derived from experience values of a particular dialyzer.

The effective urea clearance, determined by other methods known in the art, can also be used for the transport rate Tr, since it has been found to be very similar to effective ionic dialysance.

According to another feature of the invention as in claim 4 it may be provided to that, during said time interval T the control unit (85) acts on said switching means to carry out the following consecutive sub-steps:

a. First configuring the said arterial and venous lines according to the normal configuration for obtaining said first concentration or first conductivity (Cn), and then
b. configuring the arterial and venous lines according to the reversed configuration for obtaining said second concentration or conductivity (Cr),
c. returning the arterial and venous lines to the normal configuration for prosecution of the blood treatment.

Alternatively, as in claim 5,during said time interval T the control unit (85) acts on said switching means to carry out the following consecutive sub-steps:

a. First, configuring the said arterial and venous lines according to the reversed configuration for obtaining said first post treatment unit concentration or conductivity (Cr), and then
b. configuring the arterial and venous lines according to the normal configuration for obtaining said second concentration or conductivity (Cn) and then prosecuting the blood treatment.

Thanks to this alternative option it is possible to first configure the lines in the reversed configuration for the execution of the Qa determination. As for Qa calculation, a measurement in the normal configuration is also necessary, by starting in reversed configuration and then passing to normal configuration there is no risk to leave the lines in reverse configuration which would lead to a reduced treatment efficiency.
Another advantage with this modified apparatus is that we have an automatic indication that the lines have actually been returned to normal for the rest of the treatment, otherwise there will be no access flow measurement. With the original procedure it is much more difficult for the machine to detect if the lines are left in the reversed position for the rest of the treatment.
In term of fistula flow determination, notice that two things will happen if we go from reversed lines back to normal instead of the other way around. First of all, the clearance measured at the conductivity change will be a clearance with reversed lines. This clearance is lower than the normal clearance, how much is determined by the access flow rate. Secondly, the conductivity change caused by returning the lines to normal will go in the opposite direction to normal. The sign of the conductivity change can be handled just by using the absolute value of the change, but the lower clearance value needs to be handled by a change in the formula. As the access flow rate (A) depends on normal configuration clearance ($K_n$), ultrafiltration rate (UF) and reversed flow configuration clearance ($K_r$) according to

$$A = \left(K_n - UF\right) \cdot \left(\frac{K_r}{K_n - K_r}\right) \qquad (1)$$

then

$$A = \left(K_n - UF\right) \cdot R \qquad (2)$$

with R determined from the inlet conductivity ($C_i$), and the outlet conductivities in normal ($C_n$) and reversed ($C_r$) positions according to

$$R = \left(\frac{c_r - c_i}{c_n - c_r}\right) \qquad (3)$$

Combining (1) and (2) we see that

$$K_n \cdot R = K_r \cdot R + K_r \qquad (4)$$

Access flow rate can therefore be calculated as

$$A = \left(K_n - UF\right) \cdot R = K_r \cdot R + K_r - UF \cdot R = \left(K_r - UF\right) \cdot R + K_r \qquad (5)$$

Since $K_r$ is the measured clearance when the lines are reversed, the only modification to the formula for access flow that has to be made if the lines are reversed from the beginning is that we must add the measured clearance. Note however for the calculation of R that $C_n$ and $C_r$ will switch positions time wise if the lines are reversed from the start (i.e. $C_r$ will be measured before $C_n$).

Note that in the present description and in the claims $C_n$ refers always to conductivity-concentration of the effluent dialysis fluid in normal configurations of the lines while $C_r$ refers always to conductivity-concentration of the effluent dialysis fluid in reversed configuration of the lines. If the time sequence adopted is first reversed then normal configuration: the first post treatment unit conductivity-concentration of the dialysis liquid is $C_r$ while the second post treatment unit conductivity-concentration is $C_n$. If the time sequence adopted is first normal then reversed configuration: the first post treatment unit conductivity-concentration of the dialysis liquid is $C_n$ while the second post treatment unit conductivity-concentration is $C_r$.

During running of the above-disdosed apparatus, the post dialyser treatment unit conductivities (first and second) are measured after a delay allowing equilibrium to establish.

According to a feature of the invention, the post dialyser treatment unit conductivity after the flow reversal is measured at various intervals or continuously so that the value of the conductivity at the time of the flow reversal can be determined by extrapolating the measured values backwards to the moment of the flow reversal. In this way the apparatus can compensate for drift of parameters between the time when the flow is reversed until the time where a substantial equilibrium is reached.

BRIEF DESCRIPTION OF THE DRAWINGS

In the following detailed portion of the present description, the invention will be explained in more detail with reference to the exemplary embodiments shown in the drawings, in which

Fig. 1 is a partially schematic view of a forearm of a patient provided with an AV fistula.
Fig. 2 is a schematic diagram of an extracorporeal circuit and part of the fluid path of a dialysis machine.
Fig. 3 is a schematic diagram of an extracorporeal circuit including a flow reversal valve.
Fig. 4 is the schematic diagram of Fig. 3, with the valve turned for reversed blood flow
Fig. 5 is a graph showing the conductivities before and after flow reversal, and
Fig. 6 is another graph showing the conductivities before and after flow reversal.

DESCRIPTION OF DETAILED EMBODIMENTS OF THE INVENTION

For the purpose of this description, a blood access is a site in which a fluid in a tube can be accessed and removed from and/or returned to the tube. The tube may be a blood vessel of a mammal, or any other tube in which a fluid is flowing. The general term blood access as used here includes arterio-venous fistulas, arterio-venous grafts, and dual-lumen catheters amongst other similar types of blood access that allow for an upstream access position and a downstream access position.
The general terms dialyzer or blood treatment unit as used here include filters for hemodialysis, hemofilters, hemodiafilters, plasmafilters and ultrafilters.
The fluid flow rate is the flow rate of the fluid in the tube or blood vessel immediately upstream of the blood access, denoted Qa.
The general term dialysis as used here includes hemodialysis, hemofiltration, hemodiafiltration and therapeutic plasma exchange (TPE), among other similar treatment procedures.
The general term effluent fluid as used here refers to the dialysis fluid downstream of the dialyzer or blood treatment unit.
The general term "transport of substances or ions though the semi permeable membrane" includes any parameter that is indicative of the rate at which substances or ions pass through the dialyzer membrane. Examples of such parameters are, clearance, urea clearance, dialysance, ionic dialysance and effective ionic dialysance.
The general term ionic dialysance as used here refers to a variable that expresses the transport of ions through the dialyzer membrane. The ionic dialysance is ion dependent, i.e. different ions have different dialysance values. It is also dependent on blood flow, dialysate flow and Quf, so during measurements when determining the access flow these must preferably be held constant. The effective ionic dialysance, herein denoted D, further depends on recirculation effects in the fistula and the cardiopulmonary circuit, and is obtained for example as described by EP 658 352. The major ions determining the conductivity of dialysate liquid are sodium and chloride

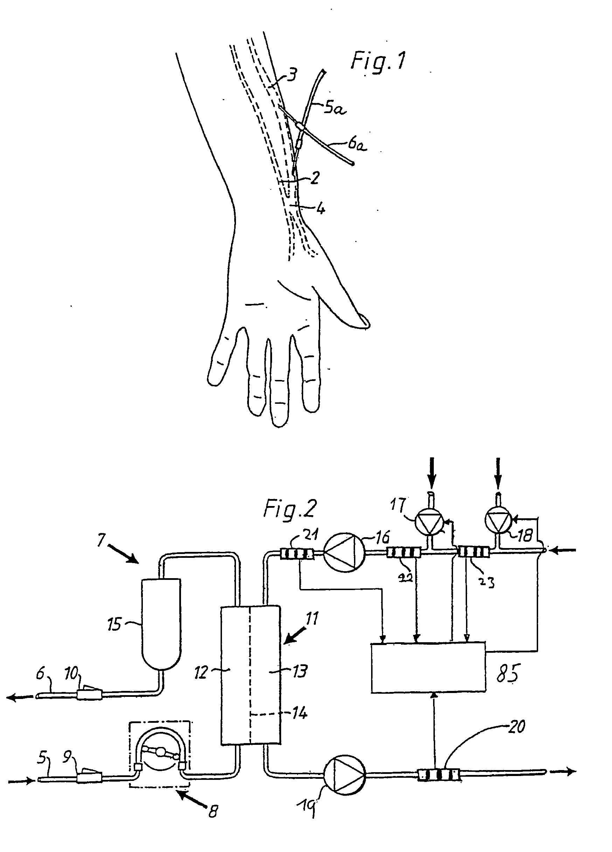

Fig. 1 discloses a forearm 1 of a human patient. The forearm 1 comprises an artery 2, in this case the radial artery, and a vein 3, in this case the cephalic vein. Openings are surgically created in the artery 2 and the vein 3 and the openings are connected to form a fistula 4, in which the arterial blood flow is cross-circuited to the vein. Due to the fistula, the blood flow through the artery and vein is increased and the vein forms a thickened area downstream of the connecting openings. When the fistula has matured after a few months, the vein is thicker and may be punctured repeatedly. Normally, the thickened vein area is called a fistula.

An arterial needle 5a, to which is connected a piece of tube, is placed in an upstream position in the fistula, in the enlarged vein close to the connected openings and a venous needle 6a, to which is connected a piece of tube, is placed in a position downstream of the arterial needle, normally at least five centimeters downstream thereof.

As described above, the blood access can also be an arterio-venous graft, a double lumen catheter or other similar arrangements.

The needles 5a and 6a are connected to a tube system, shown in Fig. 2, forming an extracorporeal circuit 7 comprising a blood pump 8, such as a peristaltic pump. The blood pump propels blood from the fistula, through the arterial needle, the extracorporeal circuit, the venous needle, and back into the fistula.

The extracorporeal blood circuit 7 shown in Fig. 2 further comprises an arterial clamp 9 and a venous clamp 10 for isolating the patient from the extracorporeal circuit should an error occur.

Downstream of pump 8 is a dialyzer 11, comprising a first, so called blood chamber 12 and a second, so called dialysis fluid chamber 13 separated by a semi permeable membrane 14. Further downstream of the dialyzer is a drip chamber 15, separating air from the blood therein.

The bloodline upstream of the dialyzer 11 is referred to as the arterial line 5, whereas the bloodline downstream from the dialyzer 11 is known as the venous line 6. The arterial and venous lines 5 and 6 are able to be configured according to at least a normal configuration, in which said arterial line carries blood from said upstream position of said blood access and said venous line carries blood towards said downstream position of said blood access, and to at least a reversed configuration, in which said arterial line carries blood from said downstream position of said blood access and said venous line carries blood towards said upstream portion of said blood access.

In the normal configuration, blood passes from the arterial needle past the arterial clamp 9 to the blood pump 8. The blood pump drives the blood through the dialyzer 11 and further via the drip chamber 15 and past the venous clamp 10 back to the patient via the venous needle. The drip chamber may comprise an air detector, adapted to trigger an alarm should the blood emitted from the drip chamber comprise air or air bubbles. The blood circuit may comprise further components, such as pressure sensors etc.

The dialysis fluid chamber 13 of the dialyzer 11 is provided with dialysis fluid via a first pump 16, which obtains dialysis fluid from a source of pure water, normally RO-water, mixed with one or several concentrates of ions, varying means including metering pumps 17 and 18 being shown for metering such concentrates. Sensors comprising aA conductivity cell 22 and a conductivity cell 23 are provided downstream of the points where the concentrates are mixed into the main fluid steam. The signal of the respective conductivity cell 22,23 is in a closed loop manner compared with the desired conductivity and the speed of the pumps 17 and 18 are controlled in response. A further conductivity cell 21, connected to the protective system of the dialysis machine, is provided downstream from all concentrate mixing steps measuring the final total conductivity. The protective system compares the measured final conductivity with a calculated final conductivity and puts the dialysis machine in a safe state, if anything should have gone wrong in the mixing steps.

A control unit 85 operates said varying means for circulating a dialysis liquid in the second chamber of said treatment unit in such a way that, at least for a time interval T, said dialysis liquid upstream the treatment unit has a concentration (Ci) of one or more substances different from the concentration of the same substance(s) in blood.

According to an embodiment of the invention the difference in concentration is measured as a difference in the conductivity, because most of the components in the dialysis liquid are electrolytes and thus a change in their concentration will inherently lead to a change in the conductivity of the dialysis liquid. It will be understood though, that the invention can also be carried out using the concentration of substances that have no or little effect on the conductivity of the liquid that they are dissolved in, such as urea or glucose.

A preferable range for the dialysate conductivity during the blood access flow measurement is 14,5 to 17,5 mS/cm, preferably about 15 to 16 mS/cm. Thus a conductivity difference between the blood and the dialysate of about 1 to 2 mS/cm is created.

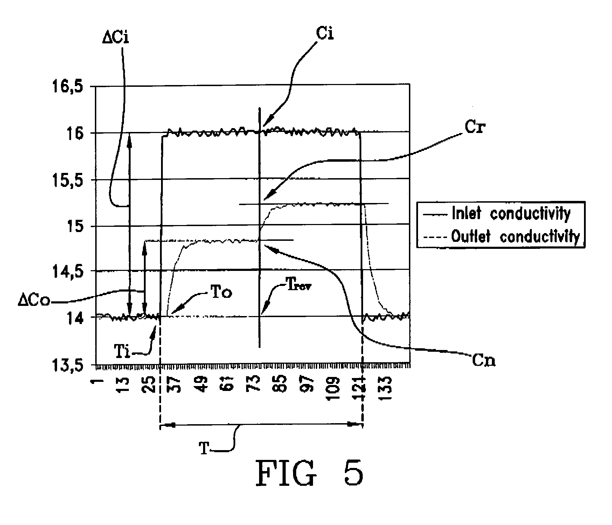

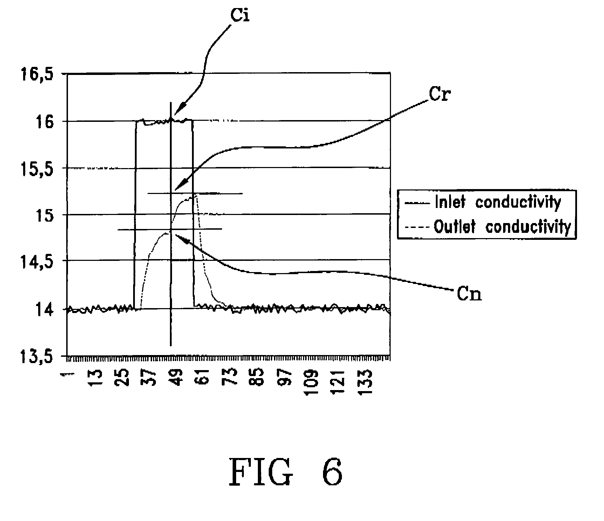

In the specific embodiment shown in figures 5 and 6 an increase in conductivity (concentration of one or more electrolytes) is applied to the fluid upstream the second chamber 13. Said increase starts at time Ti in order to bring the second chamber inlet conductivity to a substantially constant value Ci for a certain time interval T.

According to a first alternative, the invention can work even if instead of an increase a decrease in conductivity or concentration is applied to the fluid at the inlet of the second chamber.

According to a second alternative, if the dialysis liquid inherently has the required difference in conductivity with respect to the blood, then no change in conductivity shall be created for performing the method according to the invention.

A major contribution to the conductivity of the dialysis liquid is sodium chloride. From a physiological standpoint and for best control, the preferred way to adjust the final total conductivity is therefore to change the concentration of sodium chloride. The control unit 85 changes the setting of sodium chloride and in response the speed of metering pump 17 and/or 18 is adjusted as described above. In many types of dialysis apparatus however, the sodium chloride is in a concentrate container together with all the minor amounts of other electrolytes e.g. potassium, magnesium, calcium and peracetic acid, the so called "A concentrate". This concentrate contributes about 12 mS/cm of the usual final 14 mS/cm conductivity. The remainder of the conductivity comes from the bicarbonate concentrate. In such a dialysis machine (not shown) the conductivity is set by changing the amount of A concentrate in the same way as described above for sodium chloride alone.

Though less attractive from a physiological point of view, it is also possible to change the concentration of all electrolytes, i.e. inclusive bicarbonate simultaneously. It is also possible to change the concentration of any other electrolytes or other components such as glucose.

An exchange of substances between the blood and the dialysis fluid takes place in the dialyzer 11 through the semi permeable membrane 14. The exchange may take place by diffusion under the influence of a concentration gradient, so called hemodialysis, and/or by convection due to a flow of liquid from the blood to the dialysis fluid, so called ultrafiltration.

From the dialysis fluid chamber 13 of the dialyzer is emitted a fluid called the effluent fluid, which is driven by a second pump 19 via a conductivity cell 20 to drain. The conductivity cell measures continuously or at various intervals, the conductivity of the effluent fluid emitted from the dialyzer, to provide an effluent fluid conductivity.

As described above, the present invention provides an apparatus for non-invasively measuring the fluid flow in the fistula immediately before the arterial needle, using the conductivity cell 20 and the dialysis circuit as shown in Fig. 2.

By measuring the first post dialyzer liquid conductivity-concentration during normal dialysis (or normal configuration of the venous and arterial lines) and then reversing the positions of the needles (reversed configuration not shown) or any other suitable way to perform a flow reversal and measuring the second post dialyzer conductivity-concentration with the needles in the reversed position, the control unit is able to it is possible to calculate the blood flow in the blood access, without the addition of any substance to the blood or the dialysis fluid solely for the sake of the measurement.

Note that in order to pass from the normal configuration of the lines to the reversed configuration of the lines the following alternative options can be used.

Various other ways for achieving the flow reversal are known to the skilled person.

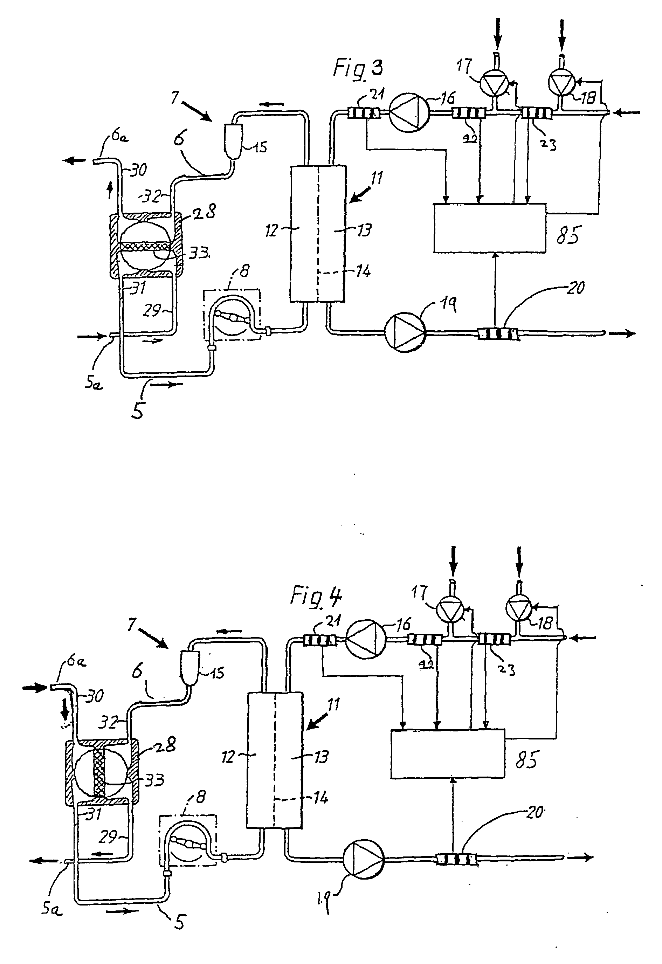

Another embodiment usable for switching the lines between the normal and the reversed condition and viceversa is shown in Figs. 3 and 4. These figures relate to show a schematic diagram of the dialysis circuit according to Fig. 2 with the addition of a valve 28 to perform the flow reversal. The arterial needle 5a is connected to an arterial inlet line 29 of the valve and the venous needle 6a is connected to a venous inlet line 30 of the valve. The blood pump is connected via arterial line 5 to a first outlet line 31 of the valve and the blood returning from the dialyzer 11 is connected via the venous line 6 to a second outlet line 32 of the valve. The valve 28 comprises a valve housing and a pivotable valve member 33, which is pivotable from the normal position shown on the drawing to a reverse position pivoted 90° in relation to the normal position. In the normal position shown in Fig. 3, the arterial needle 5a is connected to the blood pump 8 and the venous needle 6a is connected to the outlet of the dialyzer, via the drip chamber 15. In the reversed position shown in Fig. 4, the arterial needle 5a is connected to the outlet of the dialyzer and the venous needle 6a is connected to the blood pump 8, as required. Thus the flow is "reversed", and the arterial line 5 carries blood from a downstream position of the blood access, and the venous line 6 carries blood towards an upstream position of the blood access. According to an embodiment, the dialysis machine automatically controls the change of the valve position.

As mentioned before other systems may be used to pass form a configuration to the other; for instance manually changeable connections in the arterial line to the downstream position of the blood access and in the venous line to an upstream position of the blood access.

Alternatively the lines may be designed to present first conduits connecting the arterial line to both the upstream and the downstream position of the blood access and second conduits connecting the venous line to both the upstream and the downstream position of the blood access. In order to operate the configuration, means for selectively closing one of the first conduits between the arterial line and the blood access and means for selectively closing one of the conduits between the venous line and the blood access can be provided. Such closing means can be manually operable valves or valves controlled by the blood treatment apparatus. Pinch valves, cam valves or clamps having portions active on respective tube portions can be used.

As a further alternative flow distribution means can be used able of connecting the arterial line with the upstream position of the access point and the venous line with the downstream position of the access point, in a first state of said flow distribution means, and able to connect the arterial line with the downstream position of the access point and the venous line with the upstream position of the access point, in a second state of said flow distribution means.

Figures 5 and 6 are graphs of measured pre and post dialyzer conductivities. The horizontal axis represent the lapsed times and the vertical axis represent the measured conductivity in mS/cm. In figures 5,6 it is assumed to start with the venous and arterial lines in normal condition and to switch the lines into the reversed condition during the time interval T of change of the conductivity of the dialysis fluid. As already mentioned it is possible to execute the method according to the invention starting with the reversed condition.

For determining the fluid flow rate in the blood access, a gradient between the conductivity of the dialysis fluid (Ci) at the dialyzer inlet and the blood (Cb) is created (Fig. 5). Hereto the conductivity of the dialysis liquid is increased from the conventional value of 14 mS/cm (first dialysis liquid having conductivity which corresponds roughly to the conductivity of blood) to 16 mS/cm (second dialysis liquid). The difference may be of another magnitude and, as already mentioned, can also be created by reducing the conductivity of the dialysis fluid. The conductivity of the second liquid is at least 2mS/cm (2 milli-Siemens / centimeter) higher than the conductivity of the first liquid if the conductivity of the first liquid is less or equal to 15mS/cm.

The conductivity gradient is preferably obtained by changing the sodium chloride concentration, but may also be obtained by varying the concentrations of any of the other electrolytes present in dialysis fluid. The change in electrolyte concentration can in advanced dialysis machines such as the Gambro AK 200 S® be executed by changing the settings or programming a step through the user interface. Use of conductivities instead of concentrations is simpler, more reliable, cheaper to implement as it employs the conventional sensors of the treatment apparatus, does not need determination of D or K in two different conditions.

In Figs. 5 and 6 the conductivity of the dialysis fluid Ci prepared by the dialysis monitor is increased from 14 to 16 mS/cm at time Ti. The conductivity Cn of the post dialyzer fluid, the effluent fluid, will begin to increase at time To with a delay To-Ti caused by the volume of the tubes and the dialyzer. Cn will reach a semi stable value only after some time. Because the increased conductivity of the dialysis liquid causes a transport of ions form the dialysis liquid to the blood, which therefore also slowly increases in conductivity, there will be a slow drift in of the post dialyzer conductivity. The value of Cn may be determined after the respective value has become substantially stable, as shown in Figure 5. In order to further improve the precision of the method the value of Cn may be extrapolated forward to the point in time of the flow reversal $T_{rev}$. Alternatively, the value of Cn may be determined while it is still increasing by estimating which substantially stable value Cn would have reached after an equilibrium has been established by using numerical methods such as curve fitting or and/or extrapolation, in order to determine the value of Cn at $T_{rev}$, shown in Figure 6. The latter approach will allow the method to be carried out in a shorter time span.

The next step is to reverse the flow at $T_{rev}$ (cf. Figs. 5 and 6) as described above, i.e. a blood flow in a second direction is created in which the venous line 6 carries treated blood from the dialyzer 11 via arterial needle 5a to the upstream position of the blood access. The arterial line 5 draws in blood from the downstream position via venous needle 6a towards the dialyzer 11.

The effect of this measure is a further increase in the effluent conductivity, which after the flow reversal is referred to as Cr. Cr will reach a semi stable value only asymptotically. The value of Cr may be determined after it has become substantially stable, as shown in Figure 5. The value of Cr may be extrapolated backwards to the point in time of the flow reversal $T_{rev}$. Alternatively, the value may be determined while the conductivity is still increasing by estimating which substantially stable value Cr would have reached at $T_{rev}$ after an equilibrium has been established by using numerical methods such as curve fitting or extrapolation, as shown in figure 6.

The volumes in the dialyzer and connecting tubes that need to be exchanged cause the delay. During the delay period, changes in other parameters may occur and could influence the measurement negatively. The preferred method uses therefore the values extrapolated, to the point in time where the flow reversal took place. The above techniques allow estimating the value of Cn and of Cr at the same time Tr, thereby increasing the accuracy in Qa calculation.

Unit 85 may then calculate the fluid flow rate in the blood access in accordance with the formula:

$$Qa = (Tr - Quf) \cdot (Cr - Ci)/(Cn - Cr),$$

wherein:

Qa = fluid flow rate in the blood access
Tr = transport rate of substances through the semipermeable membrane
Ci = dialysis liquid conductivity upstream the treatment unit or dialyzer 11
Cn = effluent conductivity referring to the dialysis liquid before flow reversal
Cr = effluent conductivity referring to the dialysis liquid after flow reversal
Quf = ultrafiltration flow rate (Quf).

The transport rate may be based on experience values of a particular dialyzer, such as the clearance, calculated from dialyzer capacity and flow rates or measured by comparing a pre-dialysis blood sample with an initial dialysis liquid urea concentration. Alternatively the transport rate (Tr) corresponds to measured effective ionic dialysance (D) or to measured clearance K of the dialyzer, preferably the urea clearance value. The ultrafiltration flow rate Quf is on conventional dialysis machines continuously measured and monitored. The equation can therefore be solved and the fluid flow rate in the blood access is determined.

Alternatively to what described above with reference to figures 5,6, the measurement of Qa may be obtained by first configuring the lines in the reversed configuration. Then a change in conductivity or concentration (for instance by means of a step increase or decrease in the concentration of defined solutes in the dialysis liquid) is created and finally the concentration or conductivity of the dialysis liquid downstream the dialyzer is measured both for the liquid in reversed condition and for the liquid in normal condition. This second approach is convenient if the Qa measurement is carried out at the beginning of the dialysis session. Indeed the patient can be first connected to the treatment apparatus with the lines in reversed configuration; then when necessary the lines are reversed, the Qa calculated and the treatment can prosecute normally at high efficiency with no need of further line switching as the line are already in normal configuration.

In case the apparatus is run starting from the reversed configuration, then the Qa is still calculated as a function of the above-identified parameters.

If Tr is determined from the measured clearance K or the measured effective ionic dialysance D in vivo values obtained when said venous and arterial lines are in the normal configuration, the fluid flow rate (Qa) in said blood access is calculated by the formula $Qa=(Tr-Quf)*(Cr-Ci)/(Cn-Cr)$, where Tr is the transport rate when the lines are in the normal configuration.

If Tr is obtained from the measured clearance K or the measured effective ionic dialysance D in vivo values obtained when said venous and arterial lines are in the reversed configuration, the fluid flow rate (Qa) in said blood access is calculated by the formula $Qa=(Tr_r-Quf)*(C_r-Ci)/(C_n-C_r)+Tr_r$, where $Tr_r$ is the transport rate when the lines are in the reversed configuration.

The measured clearance K or the measured effective ionic dialysance D in vivo values can obtained by the following steps:

a. passing a third dialysis liquid through the second chamber of said treatment unit, said dialysis liquid presenting a concentration for at least one substance, then
b. obtaining a third post treatment unit conductivity of the dialysis liquid or third post treatment unit concentration of said substance for the third dialysis liquid,
c. at least for a second time interval , increasing or decreasing the concentration of the substance in the third dialysis liquid for passing a fourth liquid through the second chamber inlet, said fourth liquid having a concentration of at least said substance different from the concentration of the same substance in the third liquid,
d. obtaining a fourth post treatment unit conductivity of the dialysis liquid or fourth post treatment unit concentration of said substance for the fourth dialysis liquid, calculating the in vivo value of K or D as a function of said third post treatment unit concentration or conductivity and of said fourth post treatment unit concentration or conductivity.

In particular the measured clearance K or the measured ionic dialysance D can be determined during the time interval T so as to use the change in conductivity necessary for the implementation of the present invention. In this case a separate modification of the liquid arriving at the second chamber 13 is not necessary and the third liquid corresponds to the first liquid (before the step in figures 5,6) and the fourth liquid corresponds to the second liquid (after the step in figures 5,6).

Practically if only ions concentration is altered, and again referring to the example of figure 5,

$$Tr \approx K$$

$$K = (D+U) \cdot (1 - \frac{\Delta C_o}{\Delta C_i}) \qquad \frac{\Delta C_o}{\Delta C_i}$$

being the inverse of the rate between the step in conductivity of the dialysis fluid at the dialyser inlet and the corresponding step of the dialysis liquid at the outlet of the dialyzer

$$A = (K - U) \cdot \left( \frac{C_i - C_r}{C_r - C_n} \right)$$

The upstream conductivity cell should preferably calibrated relative to the downstream conductivity cell 20 for improved accuracy. Preferably temperature compensated conductivity cells are used to improve the accuracy of the method.

The value for Ci may be determined by measuring the conductivity of the dialysis fluid before it enters the dialyzer. Alternatively the set value for the dialysis fluid conductivity may be used, since the actual conductivity will only differ marginally from the set value as dialysis monitors control the conductivity of the dialysis fluid very accurately.

**Claims**

1.  A blood treatment apparatus for determining the fluid flow rate (Qa) in a blood access having a downstream position and an upstream position, the apparatus comprising: a dialysis liquid source,

    a. a treatment unit (11), having a semi permeable membrane (14) delimiting a first chamber (12) through which blood removed from said blood access passes and a second chamber(13) through which dialysis liquid passes,
    b. a dialysis liquid line for circulating dialysis liquid in the second chamber (13);
    c. an arterial line (5) connected to an inlet of the first chamber (12),
    d. a venous line (6) connected to an outlet of the first chamber (12),
    e. said arterial and venous lines (5, 6) being able to be configured according to at least a normal configuration, in which said arterial line (5) carries blood from said upstream position of said blood access, and said venous line (6) carries blood towards said downstream position of said blood access, and to at least a reversed configuration, in which said arterial line (5) carries blood from said downstream position of said blood access, and said venous line (6) carries blood towards said upstream position of said blood access,
    f. means for switching (28) the venous and arterial lines (5, 6), during a time interval T, between one of said normal and reversed configurations to the other of said normal and reversed configurations,
    g. means for varying (17, 18) a concentration (Ci) of at least a substance of the dialysis liquid upstream the treatment unit;
    h. a sensor (20) operating downstream the treatment unit (11) for detecting a post-treatment unit conductivity of the dialysis liquid or a post treatment unit concentration of said substance in the dialysis liquid, and
    i. a control unit (85) capable of performing the following steps:

    o operating said varying means (17, 18) in such a way that, at least for said time interval T, said dialysis liquid circulating upstream the second chamber of the treatment unit (11) comprises at least a substance having a concentration (Ci) different from the concentration of the same substance in blood,
    o obtaining from said sensor (20) a first post-treatment unit conductivity of the dialysis liquid or a first post treatment unit concentration of said substance in the dialysis liquid, for the venous and arterial lines (5, 6) being configured according to one of said normal or reversed configuration, said first conductivity or concentration relating to the dialysis liquid before switching the venous and arterial lines (5, 6) and during said time interval T;
    o obtaining, from said sensor (20) a second post treatment unit conductivity of the dialysis liquid or post treatment unit concentration of said substance in the dialysis liquid, for the venous and arterial lines (5, 6) being configured according to the other of said normal or reversed configuration, said second conductivity or concentration relating to the dialysis liquid after switching of the venous and arterial lines and during said time interval T;
    o calculating the fluid flow rate (Qa) in said blood access as a function of said first post treatment unit concentration or conductivity and of said second post treatment unit concentration or conductivity,

    **Characterized in that** the control unit (85) is capable of acting on the varying means (17, 18) to keep substantially constant the concentration (Ci) of said at least a substance of the dialysis liquid upstream the treatment unit during said time interval T.

2.  Apparatus according to claim 1, wherein said sensor comprises a post treatment unit conductivity (Cn,Cr) cell.

3. Apparatus according to claim 1, wherein the varying means are designed for increasing or decreasing the concentration of one or more substances in the dialysis liquid.

4. Apparatus according to claim 1, wherein during said time interval T the control unit (85) acts on said switching means to carry out the following consecutive sub-steps:

   a. First configuring the said arterial and venous lines according to the normal configuration for obtaining said first concentration or first conductivity (Cn), and then
   b. configuring the arterial and venous lines according to the reversed configuration for obtaining said second concentration or conductivity (Cr),
   c. returning the arterial and venous lines to the normal configuration for prosecution of the blood treatment.

5. Apparatus according to claim 1, wherein during said time interval T the control unit (85) acts on said switching means to carry out the following consecutive sub-steps:

   a. First, configuring the said arterial and venous lines according to the reversed configuration for obtaining said first post treatment unit concentration or conductivity (Cr), and then
   b. configuring the arterial and venous lines according to the normal configuration for obtaining said second concentration or conductivity (Cn) and then prosecuting the blood treatment.

6. Apparatus according to claim 1, wherein the control unit (85) is able to perform a step of checking if the arterial and venous lines are in said normal or in said reversed configuration.

7. Apparatus according to claim 4, wherein after having configured the arterial and venous lines according to the reversed configuration the control unit is able to perform a further step of checking if the arterial and venous lines are in said first or in said reversed configuration.

8. Apparatus according to claim 1, wherein the control unit (85) is able to perform the following steps:

   a. determining the transport rate (Tr) of ions though the semi permeable membrane,
   b. obtaining the first post treatment unit conductivity (Cn, Cr) relating to the dialysis liquid before switching the venous and arterial lines,
   c. obtaining the second post treatment unit conductivity (Cr, Cn) relating to the dialysis liquid after switching the venous and arterial lines,
   d. calculating the fluid flow rate (Qa) in said blood access as a function of said first and second post treatment unit conductivities and of said transport rate.

9. Apparatus according to claim 8, wherein the fluid flow rate (Qa) is calculated from the values of said transport rate (Tr), said first post treatment unit conductivity (Cn,Cr), said second post treatment unit conductivity (Cr,Cn), and the conductivity of the dialysis liquid (Ci) upstream the treatment unit.

10. Apparatus according to claim 8, wherein said first and second post treatment unit conductivities (Cn, Cr) are obtained by said sensor.

11. Apparatus according to anyone of the claims 1 to the preceding claim, further comprising means acting on the dialysis line for causing an ultrafiltration flow rate (Quf).

12. Apparatus according to claim 9 or 11, wherein the fluid flow rate (Qa) in said blood access is calculated by the formula $Qa=(Tr)*(Cr-Ci)/(Cn-Cr)$ or by the formula $Qa=(Tr-Quf)*(Cr-Ci)/(Cn-Cr)$, wherein Tr is determined from the measured clearance K or the measured ionic dialysance D in vivo values obtained when said venous and arterial lines are in the normal configuration.

13. Apparatus according to claim 9 or 11, wherein $Tr_r$ is the transport rate obtained from the measured clearance K or the measured ionic dialysance D in vivo values obtained when said venous and arterial lines are in the reversed configuration, the fluid flow rate (Qa) in said blood access being calculated by the formula $Qa=(Tr_r -Quf)*(Cr-Ci)/(Cn-Cr)+ Tr_r$.

14. Apparatus according to any of the claims 1 to the preceding claim, further comprising means for preparing dialysis liquid with a conductivity different from said blood, preferably said means comprises means for controlled mixing of electrolyte concentrates with water.

15. Apparatus according to any of the claims 1 to the preceding claim, wherein said switching means comprises:

- manually changeable connections in the arterial line to the downstream position of the blood access and in the venous line to an upstream position of the blood access, or
- first conduits connecting the arterial line to both the upstream and the downstream position of the blood access and second conduits connecting the venous line to both the upstream and the downstream position of the blood access, means for selectively closing one of the first conduits between the arterial line and the blood access and means for selectively closing one of the conduits between the venous line and the blood access, or
- a valve able to connect the arterial line with the upstream position of the access point and the venous line with the downstream position of the access point in a first position of said valve and able to connect the arterial line with the downstream position of the access point and the venous line with the upstream position of the access point in a second position of said valve, or
- flow distribution means for connecting the arterial line with the upstream position of the access point and the venous line with the downstream position of the access point in a first state of said flow distribution means and able to connect the arterial line with the downstream position of the access point and the venous line with the upstream position of the access point in a second state of said flow distribution means.

16. Apparatus according to any of the claims 1 to the preceding claim, wherein said treatment unit (11) comprises one selected in the group comprising:

i. A dialyzer;
ii. An hemofilter;
iii. A plasmafilter;
iv. An hemodiafilter;
v. An ultrafilter.

17. Software product comprising instructions executable by a control unit (85) of a blood treatment apparatus according to anyone of claims 1 to 16, said software as executed on said control unit (85) rendering it able of performing a method for determining a fluid flow rate (Qa) in a blood access having an upstream position and a downstream position using said blood treatment apparatus, comprising the steps of:

- passing a dialysis liquid through the second chamber (13) of said treatment unit (11), at least for a time interval T, said dialysis liquid upstream the treatment unit (11) comprising at least one substance having a concentration (Ci) different from the concentration of the same substance in blood,
- obtaining, downstream the second chamber (13) of said treatment unit (11), a first post-treatment unit conductivity of the dialysis liquid or a first post treatment unit concentration of said substance in the dialysis liquid, said first conductivity or concentration (Cn, Cr) relating to the venous and arterial lines configured according to one of said normal or reversed configuration, said first post treatment unit conductivity or concentration referring to the dialysis liquid before switching the venous and arterial lines and during said time interval T,
- switching the venous and arterial lines, during said time interval T, between one of said normal and reversed configurations to the other of said normal and reversed configurations,
- obtaining, downstream the second chamber (13) of said treatment unit (11), a second post treatment unit conductivity of the dialysis liquid or post treatment unit concentration of said substance in the dialysis liquid, said second conductivity or concentration (Cn, Cr) relating to the venous and arterial lines configured according to the other of said normal or reversed configuration, said second post treatment unit conductivity or concentration referring to the dialysis liquid after switching of the venous and arterial lines and during said time interval T;
- calculating the fluid flow rate (Qa) in said blood access as a function of:

o said first post treatment unit concentration or conductivity and of
o said second post treatment unit concentration or conductivity,

and wherein during said time interval T of the passage of one dialysis liquid, the concentration Ci of said at least a substance in the liquid at the treatment unit inlet is kept substantially constant.

18. Software according to claim 17, said software as executed on said control unit (85) rendering it able of performing said method wherein the step of passing a dialysis liquid through the second chamber comprises the following sub-steps:

> j. passing a first dialysis liquid through the second chamber inlet of said treatment unit, said first dialysis liquid presenting a treatment concentration for said substance, then
> k. increasing or decreasing at a time Ti the concentration of the substance in the dialysis liquid for passing through the second chamber inlet a second liquid which, during the time interval T, has a concentration of at least said substance different from the concentration of the same substance in blood.

**19.** Software according to claim 17, said software as executed on said control unit (85) rendering it able of performing said method wherein the step of passing a dialysis liquid trough the second chamber comprises the following sub-steps:

> a. passing a first dialysis liquid trough the inlet of the second chamber of said treatment unit, said first dialysis liquid presenting a treatment concentration for prefixed substances,
> b. during the time interval T, increasing or decreasing at a time Ti the concentration of more than one of said prefixed substances in the dialysis liquid for passing through the second chamber inlet a second liquid having a concentration of said substances different from the concentration of the same substances in blood.

**20.** Software according to claim 18, said software as executed on said control unit (85) rendering it able of performing said method wherein said substance is an ion.

**21.** Software according to claim 19, said software as executed on said control unit (85) rendering it able of performing said method wherein said substances are ions.

**22.** Software according to claims 19, said software as executed on said control unit (85) rendering it able of performing said method wherein the fluid flow rate (Qa) in said blood access is calculated as a function of said first post treatment unit concentrations or conductivity and of said second post treatment unit concentrations or conductivity.

**23.** Software according to claim 17, said software as executed on said control unit (85) rendering it able of performing said method wherein during said time interval T the following consecutive sub-steps are provided with:

> a. First configuring the said arterial and venous lines according to the normal configuration for obtaining said first concentration or first conductivity (Cn), and then
> b. configuring the arterial and venous lines according to the reversed configuration for obtaining said second concentration or conductivity (Cr).

**24.** Software according to claim 23, said software as executed on said control unit (85) rendering it able of performing said method comprising the step of returning the arterial and venous lines to the normal configuration for starting of a blood treatment.

**25.** Software according to claim 17, said software as executed on said control unit (85) rendering it able of performing said method wherein during said time interval T the following consecutive sub-steps are provided with:

> a. First, configuring the said arterial and venous lines according to the reversed configuration for obtaining said first post treatment unit concentration or conductivity (Cr), and then
> b. configuring the arterial and venous lines according to the normal configuration for obtaining said second concentration or conductivity (Cn).

**26.** Software according to claim 17, said software as executed on said control unit (85) rendering it able of performing said method wherein a step of checking if the arterial and venous lines are in said normal or in said reversed configuration is provided for.

**27.** Software according to claim 23, said software as executed on said control unit (85) rendering it able of performing said method wherein after having configured the arterial and venous lines according to the reversed configuration a further step of checking if the arterial and venous lines are in said normal or in said reversed configuration is provided for.

**28.** Software according to claim 26 or 27, said software as executed on said control unit (85) rendering it able of performing said method wherein the step of checking if the arterial and venous lines are in the normal or in the reversed configuration comprises the following steps:

a. Determining the in vivo value of a parameter selected in the group comprising:

i. Effective ionic dialysance D or
ii. Effective clearance K or
iii. a parameter proportional to effective ionic dialysance or
iv. a parameter proportional to effective clearance,

b. comparing the in vivo value of said parameter with a corresponding threshold value for determining if the venous and arterial lines are in said normal or in said reversed configuration.

**29.** Software according to claim 28, said software as executed on said control unit (85) rendering it able of performing said method wherein the step of determining the in vivo value of said parameter comprises the steps of:

a. passing a third dialysis liquid through the second chamber inlet of said treatment unit, said dialysis liquid presenting a concentration for at least one substance, then
b. obtaining a third post treatment unit conductivity of the dialysis liquid or third post treatment unit concentration of said substance for the third dialysis liquid,
c. at least for a second time interval , increasing or decreasing the concentration of the substance in the third dialysis liquid for passing a fourth liquid through the second chamber inlet, said fourth liquid having a concentration of at least said substance different from the concentration of the same substance in the third liquid,
d. obtaining a fourth post treatment unit conductivity of the dialysis liquid or fourth post treatment unit concentration of said substance for the fourth dialysis liquid,
e. calculating the in vivo value of said parameter as a function of said third post treatment unit concentration or conductivity and of said fourth post treatment unit concentration or conductivity.

**30.** Software according to claim 28, said software as executed on said control unit (85) rendering it able of performing said method wherein said threshold value is a set value or a calculated value or a measured value.

**31.** Software according to claim 28, said software as executed on said control unit (85) rendering it able of performing said method wherein the step of in vivo determination of said parameter is carried out during the time interval T.

**32.** Software according to claim 28, said software as executed on said control unit (85) rendering it able of performing said method wherein a further step of sending an alert signal is provided for in case the comparing step determines that the venous and arterial lines are in said reversed configuration.

**33.** Software according to claim 26, said software as executed on said control unit (85) rendering it able of performing said method wherein the step of checking if the arterial and venous lines are in said normal or in said reversed configuration is carried out during said first time interval T.

**34.** Software according to claim 17 and to claim 33, said software as executed on said control unit (85) rendering it able of performing said method wherein the step of checking if the arterial and venous lines are in said normal or in said reversed configuration comprises the following sub-steps:

- Comparing said obtained first post-treatment unit conductivity of the dialysis liquid or first post treatment unit concentration of said substance in the dialysis liquid with said obtained second post treatment unit conductivity of the dialysis liquid or second post treatment unit concentration of said substance in the dialysis liquid,
- Determining if said conductivity or concentration are increasing after the switching step.

**35.** Software according to claim 17, said software as executed on said control unit (85) rendering it able of performing said method further comprising the steps of:

a. determining the transport rate (Tr) of ions though the semi permeable membrane,
b. obtaining the first post treatment unit conductivity (Cn; Cr) for the dialysis liquid before switching the venous and arterial lines,

c. obtaining the second post treatment unit conductivity (Cr; Cn) for the dialysis liquid after switching the venous and arterial lines,

d. calculating the fluid flow rate (Qa) in said blood access as a function of said first and second post treatment unit conductivities and of said transport rate.

36. Software according to claim 18, said software as executed on said control unit (85) rendering it able of performing said method wherein the fluid flow rate (Qa) is calculated from the values of said transport rate (Tr), said first post treatment unit conductivity (Cn), said second post treatment unit conductivity (Cr), and the conductivity of the dialysis liquid (Ci) upstream the treatment unit.

37. Software according to claim 35 or 36, said software as executed on said control unit (85) rendering it able of performing said method wherein said first and second post treatment unit conductivities (Cn, Cr) are obtained by measuring the conductivity of the effluent fluid exiting from the second chamber of said treatment unit.

38. Software according to anyone of the preceding claims, said software as executed on said control unit (85) rendering it able of performing said method further comprising the step of obtaining the ultrafiltration flow rate (Quf).

39. Software according to claim 36, said software as executed on said control unit (85) rendering it able of performing said method wherein the fluid flow rate (Qa) in said blood access is calculated by the formula $Qa = (Tr)*(Cr-Ci)/(Cn-Cr)$.

40. Software according to claim 38, said software as executed on said control unit (85) rendering it able of performing said method wherein the fluid flow rate (Qa) in said blood access is calculated by the formula $Qa = (Tr-Quf)*(Cr-Ci)/(Cn-Cr)$.

41. Software according to claim 38, said software as executed on said control unit (85) rendering it able of performing said method wherein Tr is determined from the measured clearance K or the measured effective ionic dialysance D in vivo values obtained when said venous and arterial lines are in the normal configuration, the fluid flow rate (Qa) in said blood access being calculated by the formula $Qa = (Tr-Quf)*(Cr-Ci)/(Cn-Cr)$, where Tr is the transport rate when the lines are in the normal configuration.

42. Software according to claim 38, said software as executed on said control unit (85) rendering it able of performing said method wherein Tr, is transport rate of ions though the semi permeable membrane determined from the measured clearance K or the measured effective ionic dialysance D in vivo values obtained when said venous and arterial lines are in the reversed configuration, the fluid flow rate (Qa) in said blood access being calculated by the formula $Qa = (Tr_r - Quf)*(Cr-Ci)/(Cn-Cr) + Tr_r$.

43. Software according to claim 41 or claim 42, said software as executed on said control unit (85) rendering it able of performing said method wherein the measured clearance K or the measured effective ionic dialysance D in vivo values are obtained by the following steps of:

a. passing a third dialysis liquid through the second chamber (13) of said treatment unit, said dialysis liquid presenting a concentration for at least one substance, then

b. obtaining a third post treatment unit conductivity of the dialysis liquid or third post treatment unit concentration of said substance for the third dialysis liquid,

c. at least for a second time interval, increasing or decreasing the concentration of the substance in the third dialysis liquid for passing a fourth liquid through the second chamber inlet, said fourth liquid having a concentration of at least said substance different from the concentration of the same substance in the third liquid,

d. obtaining a fourth post treatment unit conductivity of the dialysis liquid or fourth post treatment unit concentration of said substance for the fourth dialysis liquid,

e. calculating the in vivo value of K or D as a function of said third post treatment unit concentration or conductivity and of said fourth post treatment unit concentration or conductivity.

44. Software according to claim 35 or 40, said software as executed on said control unit (85) rendering it able of performing said method wherein the transport rate (Tr) corresponds to:

- experience values of a particular dialyser, or
- calculated values, or
- measured effective ionic dialysance (D) or

- using a predialysis blood sample together with the initial dialysis liquid urea concentration, or
- measured clearance K of the dialyser, preferably the urea clearance value.

45. Software according to claim 41 or 42, said software as executed on said control unit (85) rendering it able of performing said method wherein the measured clearance K or the measured ionic dialysance D are in vivo values determined during the time interval T.

46. Software according to claim 45 and to claim 18, said software as executed on said control unit (85) rendering it able of performing said method wherein the third liquid corresponds to the first liquid and the fourth liquid corresponds to the second liquid.

47. Software according to claim 45 and to claim 21, said software as executed on said control unit (85) rendering it able of performing said method wherein the third liquid corresponds to the first liquid and the fourth liquid corresponds to the second liquid.

48. Software according to claim 18 or 19, said software as executed on said control unit (85) rendering it able of performing said method wherein during said time interval T the conductivity of the second liquid is higher than the conductivity of the first liquid.

49. Software according to claim 48, said software as executed on said control unit (85) rendering it able of performing said method wherein during said time interval T the conductivity of the second liquid is at least 1mS/cm (1 milli-Siemens / centimeter) higher than the conductivity of the first liquid.

50. Software according to claim 49, said software as executed on said control unit (85) rendering it able of performing said method wherein during said time interval T the conductivity of the second liquid is at least 2mS/cm (2 milli-Siemens / centimeter) higher than the conductivity of the first liquid if the conductivity of the first liquid is less or equal to 15mS/cm.

51. Software according to any of claims 48, 49, 50, said software as executed on said control unit (85) rendering it able of performing said method comprising the following steps:

> a. Changing the conductivity in the first liquid upstream the treatment unit (11) to define the second liquid,
> b. keeping substantially constant during said time interval T the conductivity of the second liquid upstream the treatment unit (11),
> c. waiting a delay after start of said conductivity change and then determine the time T0 when a prefixed change in conductivity occurs in the liquid downstream of the dialyzer (11),
> d. measuring a plurality of first values of the conductivity of the liquid downstream the treatment unit (11) after said time T0,
> e. calculating the first post-treatment unit conductivity of said liquid from said plurality of values;
> f. switching the lines from one of said configurations to the other of said configurations;
> g. measuring a plurality of second values of the conductivity of the liquid downstream the treatment unit (11) after said time switching,
> h. calculating the second post-treatment unit conductivity of said liquid from said plurality of values.

52. Software according to claim 51, said software as executed on said control unit (85) rendering it able of performing said method wherein the further step of changing the conductivity in the second liquid upstream the treatment unit (11) is provided for.

53. Software according to claim 51, said software as executed on said control unit (85) rendering it able of performing said method wherein the measurement of the first values is carried out after a delay from time T0.

54. Software according to claim 53, said software as executed on said control unit (85) rendering it able of performing said method wherein the moment Trev when the switching occurs is determined, the measurement of the second values being carried out after a delay from time Trev.

55. Software according to claim 54, said software as executed on said control unit (86) rendering it able of performing said method wherein the plurality of second values of concentration or conductivity after the switching step is continuously or intermittently measured and the concentration or conductivity (Cr) at the time of the switching $T_{rev}$

is determined by extrapolating the measured values backwards to the moment ($T_{rev}$) of the switching.

56. Software according to anyone of claims 17 to the preceding claim, said software as executed on said control unit (85) rendering it able of performing said method wherein the conductivity of the dialysis fluid is adjusted by varying the sodium chloride concentration, or by varying the concentration of all A-concentrate electrolytes simultaneously, or by varying the concentration of all electrolytes in the dialysis liquid.

57. Software product according to anyone of claims 17 to claim 56, wherein it is stored on a magnetic or optic data carrier.

58. Software product according to anyone of claims 17 to claim 56, wherein it is stored on computer memory.

59. Software product according to anyone of claims 17 to claim 56, or 57, or 58, wherein it is stored on a read only memory.

60. Software product according to anyone of claims 17 to claim 56, wherein it is stored on a computer remote from the blood treatment apparatus and is able to be transmitted on an electric or electromagnetic signal.

**Patentansprüche**

1. Blutbehandlungseinrichtung zur Bestimmung der Flüssigkeitsdurchfluss (Qa) in einem Blutzugang mit einer abwärtsliegenden Stelle und einer aufwärtsliegenden Stelle, wobei die Einrichtung umfasst: eine Dialyseflüssigkeitsquelle,

   a. eine Behandlungseinheit (11) mit einer semipermeablen Membran (14), die einen ersten Abteil (12), durch den das von dem benannten Blutzugang entfernte Blut durchfließt, und einen zweiten Abteil (13), durch den die Dialyseflüssigkeit durchfließt, definiert,
   b. eine Dialyseflüssigkeitsleitung zur Zirkulation der Dialyseflüssigkeit im zweiten Abteil (13),
   c. eine arterielle Leitung (5), die mit einem Eingang des ersten Abteils (12) verbunden ist,
   d. eine venöse Leitung (6), die mit einem Ausgang des ersten Abteils (12) verbunden ist,
   e. wobei die benannten arteriellen und venösen Leitungen (5, 6) nach mindestens einer normalen Anordnung, bei der die benannte arterielle Leitung (5) das Blut von der benannten aufwärtsliegenden Stelle des benannten Blutzugangs führt und die benannten venöse Leitung (6) das Blut zu der benannten abwärtsliegenden Stelle des benannten Blutzugangs führt, und nach mindestens einer umgekehrten Anordnung, bei der die benannte arterielle Leitung (5) das Blut von der benannten abwärtsliegenden Stelle des benannten Blutzugangs führt und die benannten venöse Leitung (6) das Blut zu der benannten aufwärtsliegenden Stelle des benannten Blutzugangs führt, konfigurierbar sind,
   f. Mittel zum Umschalten (28) der venösen und arteriellen Leitungen (5, 6) während eines Zeitintervalls T zwischen einer der benannten normalen und umgekehrten Anordnungen und der anderen der benannten normalen und umgekehrten Anordnungen,
   g. Mittel zur Änderung (17, 18) einer Konzentration (Ci) an mindestens einem Stoff der Dialyseflüssigkeit aufwärts von der Behandlungseinheit,
   h. einen Sensor (20), der abwärts von der Behandlungseinheit (11) betreibt, zur Erfassung einer Nachbehandlungseinheits-Leitfähigkeit der Dialyseflüssigkeit oder einer Nachbehandlungseinheits-Konzentration an dem benannten Stoff in der Dialyseflüssigkeit, und
   i. eine Steuereinheit (85), die die folgenden Schritte durchführen kann:

      o Betreiben der benannten Änderungsmittel (17, 18), so daß mindestens für das benannte Zeitintervall T die benannte, aufwärts von dem zweiten Abteil der Behandlungseinheit (11) zirkulierende Dialyseflüssigkeit mindestens einen Stoff mit einer anderen Konzentration als der Konzentration desselben Stoffes im Blut umfasst,
      o Erhalten von dem benannten Sensor (20) einer ersten Nachbehandlungseinheits-Leitfähigkeit der Dialyseflüssigkeit oder einer ersten Nachbehandlungseinheits-Konzentration an dem benannten Stoff in der Dialyseflüssigkeit, wobei die venösen und arteriellen Leitungen (5, 6) nach einer der benannten normalen oder umgekehrten Anordnungen konfiguriert sind, wobei die benannte erste Leitfähigkeit oder Konzentration die Dialyseflüssigkeit vor der Umschaltung des venösen und arteriellen Leitungen (5, 6) und während des benannten Zeitintervalls T betrifft,
      o Erhalten von dem benannten Sensor (20) einer zweiten Nachbehandlungseinheits-Leitfähigkeit der Dialyseflüssigkeit oder einer zweiten Nachbehandlungseinheits-Konzentration an dem benannten Stoff in der Dialyseflüssigkeit, wobei die venösen und arteriellen Leitungen (5, 6) nach der anderen der benannten normalen oder umgekehrten Anordnungen konfiguriert sind, wobei die benannte zweite Leitfähigkeit oder Konzentration die Dialyseflüssigkeit nach der Umschaltung des venösen und arteriellen Leitungen und während des benannten Zeitintervalls T betrifft,

o Berechnung der Flüssigkeitsdurchfluss (Qa) in dem benannten Blutzugang abhängig von der benannten ersten Nachbehandlungseinheits-Leitfähigkeit oder - Konzentration und von der benannten zweiten Nachbehandlungseinheits-Leitfähigkeit oder -Konzentration,

**dadurch gekennzeichnet, daß** die Steuereinheit (85) auf die Änderungsmittel (17, 18) wirken kann, um die Konzentration (Ci) an dem benannten mindestens einem Stoff der Dialyseflüssigkeit aufwärts von der Behandlungseinheit während des benannten Zeitintervalls T wesentlich <u>konstant</u> zu halten.

2. Einrichtung nach Anspruch 1, worin der benannte Sensor eine Nachbehandlungseinheits-Leitfähigkeitszelle (Cn, Cr) umfasst.

3. Einrichtung nach Anspruch 1, worin die Änderungsmittel zur Erhöhung oder Verringerung der Konzentration an einem oder mehreren Stoffen in der Dialyseflüssigkeit vorgesehen sind.

4. Einrichtung nach Anspruch 1, worin während des benannten Zeitintervalls T die Steuereinheit (85) auf die benannten Umschaltmittel wirkt, um die folgenden Unterschritte nacheinander durchzuführen:

   a. Zuerst Konfiguration der benannten arteriellen und venösen Leitungen nach der normalen Anordnung zur Erhaltung der benannten ersten Konzentration oder ersten Leitfähigkeit (Cn), und dann
   b. Konfiguration der benannten arteriellen und venösen Leitungen nach der umgekehrten Anordnung zur Erhaltung der benannten zweiten Konzentration oder Leitfähigkeit (Cr),
   c. Wiederherstellung der normalen Anordnung der arteriellen und venösen Leitungen, um die Blutbehandlungsfortzusetzen.

5. Einrichtung nach Anspruch 1, worin während des benannten Zeitintervalls T die Steuereinheit (85) auf die benannten Umschaltmittel wirkt, um die folgenden Unterschritte nacheinander durchzuführen:

   a. Zuerst Konfiguration der benannten arteriellen und venösen Leitungen nach der umgekehrten Anordnung zur Erhaltung der benannten ersten Nachbehandlungseinheits-Konzentration oder -Leitfähigkeit (Cr), und dann
   b. Konfiguration der benannten arteriellen und venösen Leitungen nach der normalen Anordnung zur Erhaltung der benannten zweiten Konzentration oder Leitfähigkeit (Cn), und dann Fortsetzung der Blutbehandlung.

6. Einrichtung nach Anspruch 1, worin die Steuereinheit (85) einen Schritt durchführen kann, bei dem es kontrolliert wird, ob die arteriellen und venösen Leitungen in der benannten normalen oder in der benannten umgekehrten Anordnung liegen.

7. Einrichtung nach Anspruch 4, worin nach der Konfiguration der arteriellen und venösen Leitungen nach der umgekehrten Anordnung die Steuereinheit einen anderen Schritt durchführen kann, bei dem es kontrolliert wird, ob die arteriellen und venösen Leitungen in der benannten ersten oder in der benannten umgekehrten Anordnung liegen.

8. Einrichtung nach Anspruch 1, worin die Steuereinheit (85) die folgenden Schritte durchführen kann:

   a. Bestimmung der ion-Förderrate (Tr) durch die semipermeable Membran,
   b. Erhaltung der ersten Nachbehandlungseinheits-Leitfähigkeit (Cn, Cr), die die Dialyseflüssigkeit vor der Umschaltung der venösen und arteriellen Leitungen betrifft,
   c. Erhaltung der zweiten Nachbehandlungseinheits-Leitfähigkeit (Cr, Cn), die die Dialyseflüssigkeit nach der Umschaltung der venösen und arteriellen Leitungen betrifft,
   d. Berechnung der Flüssigkeitsdurchfluss (Qa) in dem benannten Blutzugang abhängig von den benannten ersten und zweiten Nachbehandlungseinheits-Leitfähigkeiten und von der benannten Förderrate.

9. Einrichtung nach Anspruch 8, worin die Flüssigkeitsdurchfluss (Qa) ausgehend von den Werten der benannten Förderrate (Tr), der benannten ersten Nachbehandlungseinheits-Leitfahigkeit (Cn, Cr), der benannten zweiten Nachbehandlungseinheits-Leitfähigkeit (Cr, Cn), und der Leitfähigkeit der Dialyseflüssigkeit (Ci) aufwärts von der Behandlungseinheit berechnet wird.

**EP 1 471 957 B1**

10. Einrichtung nach Anspruch 8, worin die benannten ersten und zweiten Nachbehandlungseinheits-Leitfähigkeiten (Cn, Cr) durch den benannten Sensor erhalten werden.

11. Einrichtung nach irgendeinem der Ansprüche 1 bis zum vorherigen Anspruch, weiter umfassend Mittel, die auf die Dialyseleitung wirken, um eine Ultrafiltrationsdurchfluss (Quf) zu verursachen.

12. Einrichtung nach Anspruch 9 oder 11, worin die Flüssigkeitsdurchfluss (Qa) in dem benannten Blutzugang durch die Formel Qa = (Tr)*(Cr-Ci)/(Cn-Cr) oder durch die Formel Qa = (Tr-Quf)*(Cr-Ci)/(Cn-Cr) berechnet wird, bei der Tr ausgehend von den in-vivo gemessenen Werten der Clearance K oder der ionischen Dialysance bestimmt wird, die erhalten werden, wenn die benannten venösen und arteriellen Leitungen in der normalen Anordnung liegen.

13. Einrichtung nach Anspruch 9 oder 11, worin $Tr_r$ die Förderrate ist, die ausgehend von den in-vivo gemessenen Werten der Clearance K oder der ionischen Dialysance erhalten wird, die erhalten werden, wenn die benannten venösen und arteriellen Leitungen in der umgekehrten Anordnung liegen, wobei die Flüssigkeitsdurchfluss (Qa) in dem benannten Blutzugang durch die Formel Qa = (Tr-Quf)*(Cr-Ci)/(Cn-Cr) + $Tr_r$ berechnet wird.

14. Einrichtung nach irgendeinem der Ansprüche 1 bis zum vorherigen Anspruch, weiter umfassend Mittel zur Vorbereitung der Dialyseflüssigkeit mit einer anderen Leitfähigkeit als dem Blut, die Mittel umfassen bevorzugt Mittel zum gesteuerten Mischen der Konzentraten Elektrolyten mit Wasser.

15. Einrichtung nach irgendeinem der Ansprüche 1 bis zum vorherigen Anspruch, worin die benannten Umschaltmittel umfassen:

- manuell veränderbare Anschlüsse in der arteriellen Leitung an der abwärtsliegenden Stelle des Blutzugangs, oder
- erste Rohrleitungen, die die arterielle Leitung sowohl mit der aufwärtsliegenden Stelle als auch mit der abwärtsliegenden Stelle des Blutzugangs verbinden, und zweite Rohrleitungen, die die venöse Leitung sowohl mit der aufwärtsliegenden Stelle als auch mit der abwärtsliegenden Stelle des Blutzugangs verbinden, Mittel zum selektiven Schließen einer der ersten Rohrleitungen zwischen der arteriellen Leitung und dem Blutzugang, und Mittel zum selektiven Schließen einer der Rohrleitungen zwischen der venösen Leitung und dem Blutzugang, oder
- ein Ventil zur Verbindung der arteriellen Leitung mit der aufwärtsliegenden Stelle des Blutzugangs und der venösen Leitung mit der abwärtsliegenden Stelle des Blutzugangs in einer ersten Stellung des benannten Ventils, und zur Verbindung der arteriellen Leitung mit der abwärtsliegenden Stelle des Blutzugangs und der venösen Leitung mit der aufwärtsliegenden Stelle des Blutzugangs in einer zweiten Stellung des benannten Ventils, oder
- Flussverteilungsmittel zur Verbindung der arteriellen Leitung mit der aufwärtsliegenden Stelle des Blutzugangs und der venösen Leitung mit der abwärtsliegenden Stelle des Blutzugangs in einem ersten Zustand der benannten Flussverteilungsmittel, und zur Verbindung der arteriellen Leitung mit der abwärtsliegenden Stelle des Blutzugangs und der venösen Leitung mit der aufwärtsliegenden Stelle des Blutzugangs in einem zweiten Zustand der benannten Flussverteilungsmittel.

16. Einrichtung nach irgendeinem der Ansprüche 1 bis zum vorherigen Anspruch, worin die benannten Behandlungseinheit (11) ein Element umfasst, das auswählt ist aus der Gruppe umfassend:

i. ein Dialysegerät,
ii. einen Hämofilter,
iii. einen Plasmafilter,
iv. einen Hämodiafilter,
v. einen Ultrafilter.

17. Softwareprodukt umfassend Anleitungen, die von einer Steuereinheit (85) einer Blutbehandlungseinrichtung nach irgendeinem der Ansprüche 1 bis 16 ausführbar sind, wobei dieses Software, wenn es an dieser Steuereinheit (85) ausgeführt wird, sie ermöglicht, ein Verfahren zur Bestimmung der Flüssigkeitsdurchfluss (Qa) in einem Blutzugang mit einer aufwärtsliegenden Stelle und einer abwärtsliegenden Stelle durch die benannte Blutbehandlungseinrichtung durchzuführen, umfassend die folgenden Schritte:

- Durchdrücken einer Dialyseflüssigkeit durch den zweiten Abteil (13) der benannten Behandlungseinheit (11), mindestens für einen Zeitintervall T, wobei diese Dialyseflüssigkeit aufwärts von der Behandlungseinheit (11) mindestens einen Stoff mit einer anderen Konzentration (Ci) als die Konzentration desselben Stoffes im Blut enthält;

- Erhaltung abwärts von dem zweiten Abteil (13) der benannten Behandlungseinheit (11) einer ersten Nachbehandlungseinheits-Leitfähigkeit der Dialyseflüssigkeit oder einer ersten Nachbehandlungseinheits-Konzentration an dem benannten Stoff in der Dialyseflüssigkeit, wobei die benannte erste Leitfähigkeit oder Konzentration (Cn, Cr) die venösen und arteriellen Leitungen in einer von den benannten normalen oder umgekehrten Anordnungen betrifft, wobei die benannte erste Nachbehandlungseinheits-Leitfähigkeit oder - Konzentration die Dialyseflüssigkeit vor der Umschaltung der venösen und arteriellen Leitungen und während des benannten Zeitintervalls T betrifft,

- Umschaltung der venösen und arteriellen Leitungen während des benannten Zeitintervalls T zwischen einer von den benannten normalen oder umgekehrten Anordnungen und der anderen von den benannten normalen und umgekehrten Anordnungen,

- Erhaltung abwärts von dem zweiten Abteil (13) der benannten Behandlungseinheit (11) einer zweiten Nachbehandlungseinheits-Leitfähigkeit der Dialyseflüssigkeit oder Nachbehandlungseinheits-Konzentration an dem benannten Stoff in der Dialyseflüssigkeit, wobei die benannte zweite Leitfähigkeit oder Konzentration (Cn, Cr) die venösen und arteriellen Leitungen in der anderen von den benannten normalen oder umgekehrten Anordnungen betrifft, wobei die benannte zweite Nachbehandlungseinheits-Leitfähigkeit oder - Konzentration die Dialyseflüssigkeit nach der Umschaltung der venösen und arteriellen Leitungen und während des benannten Zeitintervalls T betrifft,

- Berechnung der Flüssigkeitsdurchfluss (Qa) in dem benannten Blutzugang abhängig von:

    o der benannten ersten Nachbehandlungseinheits-Konzentration oder - Leitfähigkeit, und
    o der benannten zweiten Nachbehandlungseinheits-Konzentration oder - Leitfähigkeit,

und worin während des benannten Zeitintervalls T des Durchflusses einer Dialyseflüssigkeit die Konzentration Ci an dem benannten mindestens einen Stoff in der Flüssigkeit an der Behandlungseinheitseingang wesentlich konstant gehalten wird.

18. Software nach Anspruch 17, wobei dieses Software, wenn es an dieser Steuereinheit (85) ausgeführt wird, sie ermöglicht, das benannte Verfahren durchzuführen, worin der Schritt des Durchflusses einer Dialyseflüssigkeit durch den zweiten Abteil die folgenden Unterschritte umfasst:

    j. Durchdrücken einer ersten Dialyseflüssigkeit durch den Eingang des zweiten Abteils der benannten Behandlungseinheit, wobei diese erste Dialyseflüssigkeit eine Behandlungskonzentration an dem benannten Stoff aufweist, dann
    k. Zunahme oder Abnahme an einer Zeit Ti der Konzentration an dem Stoff in der Dialyseflüssigkeit zum Durchdrücken durch den Eingang des zweiten Abteils einer zweiten Flüssigkeit, die während des Zeitintervalls T eine andere Konzentration an mindestens dem benannten Stoff als die Konzentration desselben Stoff im Blut aufweist.

19. Software nach Anspruch 17, wobei dieses Software, wenn es an dieser Steuereinheit (85) ausgeführt wird, sie ermöglicht, das benannte Verfahren durchzuführen, worin der Schritt des Durchdrücken einer Dialyseflüssigkeit durch den zweiten Abteil die folgenden Unterschritte umfasst:

    a. Durchdrücken einer ersten Dialyseflüssigkeit durch den Eingang des zweiten Abteils der benannten Behandlungseinheit, wobei diese erste Dialyseflüssigkeit eine Behandlungskonzentration an vorgegebenen Stoffen aufweist,
    b. Während des Zeitintervalls T Zunahme oder Abnahme an einer Zeit Ti der Konzentration an mehr als einem der benannten vorgegebenen Stoffe in der Dialyseflüssigkeit zum Durchdrücken durch den Eingang des zweiten Abteils einer zweiten Flüssigkeit, die eine andere Konzentration an den benannten Stoffen als die Konzentration derselben Stoffe im Blut aufweist.

20. Software nach Anspruch 18, wobei dieses Software, wenn es an dieser Steuereinheit (85) ausgeführt wird, sie ermöglicht, das benannte Verfahren durchzuführen, worin dieser Stoff ein Ion ist.

21. Software nach Anspruch 19, wobei dieses Software, wenn es an dieser Steuereinheit (85) ausgeführt wird, sie ermöglicht, das benannte Verfahren durchzuführen, worin diese Stoffe Ione sind.

**22.** Software nach Anspruch 19, wobei dieses Software, wenn es an dieser Steuereinheit (85) ausgeführt wird, sie ermöglicht, das benannte Verfahren durchzuführen, worin die Flüssigkeitsdurchfluss (Qa) im benannten Blutzugang abhängig von der benannten ersten Nachbehandlungseinheits-Konzentrationen oder -Leitfähigkeit und der benannten zweiten Nachbehandlungsainheits-Konzentrationen oder-Leitfähigkeit berechnet wird.

**23.** Software nach Anspruch 17, wobei dieses Software, wenn es an dieser Steuereinheit (85) ausgeführt wird, sie ermöglicht, das benannte Verfahren durchzuführen, worin während des benannten Zeitintervalls T die folgenden Unterschritte vorgesehen sind:

a. Zuerst Konfiguration der benannten arteriellen und venösen Leitungen nach der normalen Anordnung zur Erhaltung der benannten ersten Konzentration oder ersten Leitfähigkeit (Cn), und dann
b. Konfiguration der benannten arteriellen und venösen Leitungen nach der umgekehrten Anordnung zur Erhaltung der benannten zweiten Konzentration oder Leitfähigkeit (Cr).

**24.** Software nach Anspruch 23, wobei dieses Software, wenn es an dieser Steuereinheit (85) ausgeführt wird, sie ermöglicht, das benannte Verfahren durchzuführen, umfassend den Schritt der Wiederherstellung der normalen Anordnung der arteriellen und venösen Leitungen zum Starten einer Blutbehandlung.

**25.** Software nach Anspruch 17, wobei dieses Software, wenn es an dieser Steuereinheit (85) ausgeführt wird, sie ermöglicht, das benannte Verfahren durchzuführen, worin während des benannten Zeitintervalls T die folgenden Unterschritte vorgesehen sind:

a. Zuerst Konfiguration der benannten arteriellen und venösen Leitungen nach der umgekehrten Anordnung zur Erhaltung der benannten ersten Nachbehandlungseinheits-Konzentration oder -Leitfähigkeit (Cr), und dann
b. Konfiguration der benannten arteriellen und venösen Leitungen nach der normalen Anordnung zur Erhaltung der benannten zweiten Konzentration oder Leitfähigkeit (Cn).

**26.** Software nach Anspruch 17, wobei dieses Software, wenn es an dieser Steuereinheit (85) ausgeführt wird, sie ermöglicht, das benannte Verfahren durchzuführen, worin ein Schritt vorgesehen ist, bei dem es überprüft wird, ob die arteriellen und venösen Leitungen in der benannten normalen oder umgekehrten Anordnung liegen.

**27.** Software nach Anspruch 23, wobei dieses Software, wenn es an dieser Steuereinheit (85) ausgeführt wird, sie ermöglicht, das benannte Verfahren durchzuführen, worin nach der Konfiguration der arteriellen und venösen Leitungen nach der umgekehrten Anordnung ein weiterer Schritt vorgesehen ist, bei den es überprüft wird, ob die arteriellen und venösen Leitungen in der benannten normalen oder umgekehrten Anordnung liegen.

**28.** Software nach Anspruch 26 oder 27, wobei dieses Software, wenn es an dieser Steuereinheit (85) ausgeführt wird, sie ermöglicht, das benannte Verfahren durchzuführen, worin der Schritt, bei dem es überprüft wird, ob die arteriellen und venösen Leitungen in der benannten normalen oder umgekehrten Anordnung liegen, die folgenden Schritte umfasst:

a. Bestimmung des in-vivo Wertes eines Parameters, der ausgewählt wird aus der Gruppe umfassend:

i. aktuelle ionische Dialysance D oder
ii. aktuelle Clearance K oder
iii. einen Parameter, der proportional zur aktuellen ionischen Dialysance ist oder
iv. einen Parameter, der proportional zur aktuellen Clearance ist,

b. Vergleich des in-vivo Wertes dieses Parameters mit einem entsprechenden Schwellenwert zum Bestimmen, ob die venösen und arteriellen Leitungen in der benannten normalen oder umgekehrten Anordnung liegen.

**29.** Software nach Anspruch 28, wobei dieses Software, wenn es an dieser Steuereinheit (85) ausgeführt wird, sie ermöglicht, das benannte Verfahren durchzuführen, worin der Schritt der Bestimmung des in-vivo wertes des benannte Parameters die folgenden Unterschritte umfasst:

a. Durchdrücken einer dritten Dialyseflüssigkeit durch den Eingang des zweiten Abteils der benannten Behandlungseinheit, wobei diese Dialyseflüssigeit eine Konzentration an mindestens einem Stoff aufweist, dann
b. Erhaltung einer dritten Nachbehandlungseinheits-Leitfähigkeit der Dialyseflüssigkeit oder dritten Nachbehandlungseinheits-Konzentration an dem benannten Stoff für die dritte Dialyseflüssigkeit,

c. mindestens für ein zweites Zeitintervall Zunahme oder Abnahme der Konzentration an dem Stoff in der dritten Dialyseflüssigkeit zum Durchdrücken durch den Eingang des zweiten Abteils einer vierten Flüssigkeit, die eine andere Konzentration an mindestens dem benannten Stoff als die Konzentration desselben Stoffes im Blut aufweist,

d. Erhaltung einer vierten Nachbehandlungseinheits-Leiffähigkeit der Dialyseflüssigkeit oder vierten Nachbehandlungseinheits-Konzentration an dem benannten Stoff für die vierte Dialyseflüssigkeit,

e. Berechnung des in-vivo Wertes des benannten Parameters abhängig von der benannten dritten Nachbehandlungseinheits-Konzentration oder -Leitfähigkeit und der benannten vierten Nachbehandlungseinheits-Konzentration oder - Leitfähigkeit.

30. Software nach Anspruch 28, wobei dieses Software, wenn es an dieser Steuereinheit (85) ausgeführt wird, sie ermöglicht, das benannte Verfahren durchzuführen, worin der benannte Schwellenwert ein vorgegebener Wert oder ein berechneter Wert oder ein gemessener Wert ist.

31. Software nach Anspruch 28, wobei dieses Software, wenn es an dieser Steuereinheit (85) ausgeführt wird, sie ermöglicht, das benannte Verfahren durchzuführen, worin der Schritt der in-vivo Bestimmung des benannten Parameters während des Zeitintervalls T durchgeführt wird.

32. Software nach Anspruch 28, wobei dieses Software, wenn es an dieser Steuereinheit (85) ausgeführt wird, sie ermöglicht, das benannte Verfahren durchzuführen, worin ein weiterer Schritt zur Aussendung eines Alarm Signals vorgesehen ist, wenn aus dem Vergleichschritt sich ergibt, dass die venösen und arteriellen Leitungen in der benannten umgekehrten Anordnung liegen.

33. Software nach Anspruch 26, wobei dieses Software, wenn es an dieser Steuereinheit (85) ausgeführt wird, sie ermöglicht, das benannte Verfahren durchzuführen, worin der Schritt zur Überprüfung, ob die arteriellen und venösen Leitungen in der benannten normalen oder umgekehrten Anordnung liegen, während des benannten ersten Zeitintervalls T durchgeführt wird.

34. Software nach Anspruch 17 und Anspruch 33, wobei dieses Software, wenn es an dieser Steuereinheit (65) ausgeführt wird, sie ermöglicht, das benannte Verfahren durchzuführen, worin der Schritt zur Überprüfung, ob die arteriellen und venösen Leitungen in der benannten normalen oder umgekehrten Anordnung liegen, die folgenden Unterschritte umfasst:

- Vergleich der benannten ersten Nachbehandlungseinheits-Leitfilhigkeit der Dialyseflüssigkeit oder ersten Nachbehandlungseinheits-Konzentration an diesem Stoff in der Dialyseflüssigkeit mit der erhaltenen zweiten Nachbehandlungseinheits-Leitfähigkeit der Dialyseflüssigkeit oder zweiten Nachbehandlungseinheits-Konzentration an diesem Stoff in der Dialyseflüssigkeit,
- Bestimmung, ob die benannte Leitfähigkeit oder Konzentration nach dem Umschaltschritt zunehmen.

35. Software nach Anspruch 17, wobei dieses Software, wenn es an dieser Steuereinheit (85) ausgeführt wird, sie ermöglicht, das benannte Verfahren durchzuführen, weiter umfassend die folgenden Schritte:

a. Bestimmung der Ionen-Förderrate (Tr) durch die semipermeable Membran,

b. Erhaltung der ersten Nachbehandlungseinheits-Leitfähigkeit (Cn; Cr) für die Dialyseflüssigkeit vor der Umschaltung der venösen und arteriellen Leitungen,

c. Erhaltung der zweiten Nachbehandlungseinheits-Leitfähigkeit (Cr; Cn) für die Dialyseflüssigkeit nach der Umschaltung der venösen und arteriellen Leitungen,

d. Berechnung der Flüssigkeitsdurchfluss (Qa) in dem benannten Blutzugang abhängig von den benannten ersten und zweiten Nachbehandlungseinheits-Leitfähigkeiten und von der benannten Förderrate.

36. Software nach Anspruch 18, wobei dieses Software, wenn es an dieser Steuereinheit (85) ausgeführt wird, sie ermöglicht, das benannte Verfahren durchzuführen, worin die Flüssigkeitsdurchfluss (Qa) ausgehend von den Werten der Förderrate (Tr), der ersten Nachbehandlungseinheits-Leitfähigkeit (Cn), der zweiten Nachbehandlungseinheits-Leitfähigkeit (Cr), und der Leitfähigkeit der Dialyseflüssigkeit (Ci) aufwärts von der Behandlungseinheit berechnet wird.

37. Software nach Anspruch 35 oder 36, wobei dieses Software, wenn es an dieser Steuereinheit (85) ausgeführt wird, sie ermöglicht, das benannte Verfahren durchzuführen, worin die benannten ersten und zweiten Nachbehandlungseinheits-Leitfähigkeiten (Cn, Cr) durch Messung der Leitfähigkeit der Flüssigkeit, der von dem zweiten Abteil der benannten Behandlungseinheit abfließt, erhalten werden.

**38.** Software nach irgendeinem der vorherigen Ansprüche, wobei dieses Software, wenn es an dieser Steuereinheit (85) ausgeführt wird, sie ermöglicht, das benannte Verfahren durchzuführen, weiter umfassend den Schritt, bei dem die Ultrafiltrationsdurchfluss (Quf) erhalten wird.

**39.** Software nach Anspruch 36, wobei dieses Software, wenn es an dieser Steuereinheit (85) ausgeführt wird, sie ermöglicht, das benannte Verfahren durchzuführen, worin die Flüssigkeitsdurchfluss (Qa) in dem benannten Blutzugang durch die Formel Qa = (Tr)*(Cr-Ci)/(Cn-Cr) berechnet wird.

**40.** Software nach Anspruch 38, wobei dieses Software, wenn es an dieser Steuereinheit (85) ausgeführt wird, sie ermöglicht, das benannte Verfahren durchzuführen, worin die Flüssigkeitsdurchfluss (Qa) in dem benannten Blutzugang durch die Formel Qa = (Tr-Quf)*(Cr-Ci)/(Cn-Cr) berechnet wird.

**41.** Software nach Anspruch 38, wobei dieses Software, wenn es an dieser Steuereinheit (85) ausgeführt wird, sie ermöglicht, das benannte Verfahren durchzuführen, worin Tr ausgehend von den in-vivo gemessenen Werten der Clearance K oder der aktuellen ionischen Dialysance erhalten wird, die erhalten werden, wenn die benannten venösen und arteriellen Leitungen in der normalen Anordnung liegen, wobei die Flüssigkeitsdurchfluss (Qa) in dem benannten Blutzugang durch die Formel Qa = (Tr-Quf)*(Cr-Ci)/(Cn-Cr), worin Tr die Förderrate ist, wenn die Leitungen in der normalen Anordnung liegen.

**42.** Software nach Anspruch 38, wobei dieses Software, wenn es an dieser Steuereinheit (85) ausgeführt wird, sie ermöglicht, das benannte Verfahren durchzuführen, worin Tr, die Ionen-Förderrate durch die semipermeable Membran, die ausgehend von den in-vivo gemessenen Werten der Clearance K oder der aktuellen ionischen Dialysance erhalten wird, die erhalten werden, wenn die benannten venösen und arteriellen Leitungen in der umgekehrten Anordnung liegen, wobei die Flüssigkeitsdurchfluss (Qa) in dem benannten Blutzugang durch die Formel Qa = (Tr-Quf)*(Cr-Ci)/(Cn-Cr) + $Tr_r$.

**43.** Software nach Anspruch 41 oder Anspruch 42, wobei dieses Software, wenn es an dieser Steuereinheit (85) ausgeführt wird, sie ermöglicht, das benannte Verfahren durchzuführen, worin die in-vivo gemessenen Werte der Clearance K oder der aktuellen ionischen Dialysance D durch die folgenden Schritte erhalten werden:

    a. Durchdrücken einer dritten Dialyseflüssigkeit durch den zweiten Abteil (13) der benannten Behandlungseinheit, wobei diese Dialyseflüssigkeit eine Konzentration an mindestens einem Stoff aufweist, dann
    b. Erhaltung einer dritten Nachbehandlungseinheits-Leitfähigkeit der Dialyseflüssigkeit oder dritten Nachbehandlungseinheits-Konzentration an dem benannten Stoff für die dritte Dialyseflüssigkeit,
    c. mindestens für ein zweites Zeitintervall Zunahme oder Abnahme der Konzentration an dem Stoff in der dritten Dialyseflüssigkeit zum Durchdrücken einer vierten Flüssigkeit durch den Eingang des zweiten Abteils, wobei diese vierte Flüssigkeit eine andere Konzentration an mindestens dem benannten Stoff als die Konzentration desselben Stoffes im Blut aufweist,
    d. Erhaltung einer vierten Nachbehandlungseinheits-Leitfähigkeit der Dialyseflüssigkeit oder vierten Nachbehandlungseinheits-Konzentration an dem benannten Stoff für die vierte Dialyseflüssigkeit,
    e. Berechnung des in-vivo Wertes von K oder D abhängig von der benannten dritten Nachbehandlungseinheits-Konzentration oder -Leitfähigkeit und der benannten vierten Nachbehandlungseinheits-Konzentration oder -Leitfähigkeit.

**44.** Software nach Anspruch 35 oder 40, wobei dieses Software, wenn es an dieser Steuereinheit (85) ausgeführt wird, sie ermöglicht, das benannte Verfahren durchzuführen, worin die Förderrate (Tr) zu:

    - bekannten Werten eines besonderen Dialysegeräts, oder
    - berechneten Werten, oder
    - gemessener aktueller ionischer Dialysance (D), oder
    - unter Verwendung einer Vordialyse-Blutprobe zusammen mit der anfänglichen Hamstoffkonzentration in der Dialyseflüssigkeit, oder
    - gemessener Clearance K des Dialysegeräts, bevorzugt zum Wert von Harnstoff-Clearance, entspricht.

**45.** Software nach Anspruch 41 oder 42, wobei dieses Software, wenn es an dieser Steuereinheit (85) ausgeführt wird, sie ermöglicht, das benannte Verfahren durchzuführen, worin die gemessene Clearance K oder die gemessen ionische Dialysance D in-vivo, während des Zeitintervalls T bestimmte Werte sind.

**46.** Software nach Anspruch 45 und Anspruch 18, wobei dieses Software, wenn es an dieser Steuereinheit (85) ausgeführt wird, sie ermöglicht, das benannte Verfahren durchzuführen, worin die dritte Flüssigkeit zur ersten Flüssigkeit und die vierte Flüssigkeit zur zweiten Flüssigkeit entspricht.

**47.** Software nach Anspruch 45 und Anspruch 21, wobei dieses Software, wenn es an dieser Steuereinheit (85) ausgeführt wird, sie ermöglicht, das benannte Verfahren durchzuführen, worin die dritte Flüssigkeit zur ersten Flüssigkeit und die vierte Flüssigkeit zur zweiten Flüssigkeit entspricht.

**48.** Software nach Anspruch 18 oder 19, wobei dieses Software, wenn es an dieser Steuereinheit (85) ausgeführt wird, sie ermöglicht, das benannte Verfahren durchzuführen, worin während des benannten Zeitintervalls T die Leitfähigkeit der zweiten Flüssigkeit höher ist als die Leitfähigkeit der ersten Flüssigkeit.

**49.** Software nach Anspruch 48, wobei dieses Software, wenn es an dieser Steuereinheit (85) ausgeführt wird, sie ermöglicht, das benannte Verfahren durchzuführen, worin während des benannten Zeitintervalls T die Leitfähigkeit der zweiten Flüssigkeit mindestens 1 mS/cm (1 Milli-Siemens/Zentimeter) höher ist als die Leitfähigkeit der ersten Flüssigkeit.

**50.** Saftware nach Anspruch 49, wobei dieses Software, wenn es an dieser Steuereinheit (85) ausgeführt wird, sie ermöglicht, das benannte Verfahren durchzuführen, worin während des benannten Zeitintervalls T die Leitfähigkeit der zweiten Flüssigkeit mindestens 2 mS/cm (2 Milli-Siemens/Zentimeter) höher ist als die Leitfähigkeit der ersten Flüssigkeit, wenn die Leitfähigkeit der ersten Flüssigkeit unter oder gleich 15 Ms/cm beträgt.

**51.** Software nach irgendeinem der Ansprüche 48, 49, 50, wobei dieses Software, wenn es an dieser Steuereinheit (85) ausgeführt wird, sie ermöglicht, das benannte Verfahren durchzuführen, umfassend die folgenden Schritte:

a. Änderung der Leitfähigkeit in der ersten Flüssigkeit aufwärts von der Behandlungseinheit (11) zur Bestimmung der zweiten Flüssigkeit,
b. Halten der Leitfähigkeit der zweiten Flüssigkeit aufwärts von der Behandlungseinheit (11) wesentlich konstant während des benannten Zeitintervalls T,
c. Warten für eine Zeitspanne nach dem Start der Leitfähigkeitsänderung und dann Bestimmung der Zeit T0, wenn eine vorgegebene Änderung der Leitfähigkeit in der Flüssigkeit abwärts von dem Dialysegerät (11) stattfindet,
d. Messung einer Vielzahl von ersten Leitfähigkeitswerten der Flüssigkeit abwärts von der Behandlungseinheit (11) nach der Zeit T0,
e. Berechnung der ersten Nachbehandlungseinheits-Leitfähigkeit der benannten Flüssigkeit ausgehend von der benannten Vielzahl von Werten,
f. Umschaltung der Leitungen von einer der benannten Anordnungen zu der anderen der benannten Anordnungen,
g. Messung einer Vielzahl von zweiten Leitfähigkeitswerten der Flüssigkeit abwärts von der Behandlungseinheit (11) nach der Zeitumschaltung,
h. Berechnung der zweiten Nachbehandlungseinheits-Leitfähigkeit der benannten Flüssigkeit ausgehend von der benannten Vielzahl von Werten.

**52.** Software nach Anspruch 51, wobei dieses Software, wenn es an dieser Steuereinheit (85) ausgeführt wird, sie ermöglicht, das benannte Verfahren durchzuführen, worin ein anderer Schritt vorgesehen ist, bei dem die Leitfähigkeit in der zweiten Flüssigkeit aufwärts von der Behandlungseinheit (11) geändert wird.

**53.** Software nach Anspruch 51, wobei dieses Software, wenn es an dieser Steuereinheit (85) ausgeführt wird, sie ermöglicht, das benannte Verfahren durchzuführen, worin die Messung der ersten Werte nach einer Zeitspanne von der Zeit T0 durchgeführt wird.

**54.** Software nach Anspruch 53, wobei dieses Software, wenn es an dieser Steuereinheit (85) ausgeführt wird, sie ermöglicht, das benannte Verfahren durchzuführen, worin der Zeitpunkt $T_{rev}$, an dem die Umschaltung stattfindet, bestimmt wird, wobei die Messung der zweiten Werten nach einer Zeitspanne von dem Zeitpunkt $T_{rev}$ durchgeführt

EP 1 471 957 B1

wird.

**55.** Software nach Anspruch 54, wobei dieses Software, wenn es an dieser Steuereinheit (85) ausgeführt wird, sie ermöglicht, das benannte Verfahren durchzuführen, worin die Vielzahl von zweiten Werten der Konzentration oder Leitfähigkeit nach dem Umschaltschritt kontinuierlich oder intermittierend gemessen wird, und die Konzentration oder Leitfähigkeit (Cr) an dem Umschaltzeitpunkt $T_{rev}$ wird durch Extrapolation der gemessenen Werte zum Zeitpunkt ($T_{rev}$) der Umschaltung bestimmt.

**56.** Software nach irgendeinem der Ansprüche 17 bis zum vorherigen Anspruch, wobei dieses Software, wenn es an dieser Steuereinheit (85) ausgeführt wird, sie ermöglicht, das benannte Verfahren durchzuführen, worin die Leitfähigkeit der Dialyseflüssigkeit durch Änderung der Konzentration an Natriumchlorid oder durch Änderung der Konzentration an allen A-konzentrierten Elektrolyten zusammen, oder durch Änderung der Konzentration an allen Elektrolyten in der Dialyseflüssigkeit eingestellt wird.

**57.** Softwareprodukt nach irgendeinem der Ansprüche 17 bis 56, worin es auf einem magnetischen oder optischen Datenträger gespeichert ist.

**58.** Softwareprodukt nach irgendeinem der Ansprüche 17 bis 56, worin es in dem Speicher eines Computers gespeichert ist.

**59.** Softwareprodukt nach irgendeinem der Ansprüche 17 bis 56, oder 57, oder 58, worin es auf einem Nur-Lese-Speicher gespeichert ist.

**60.** Softwareprodukt nach irgendeinem der Ansprüche 17 bis 56, worin es in einem Computer mit einem Abstand von der Blutbehandlungseinrichtung gespeichert ist und an einem elektrischen oder elektromagnetischen Signal übertragen werden kann.

**Revendications**

**1.** Appareil de traitement de sang pour déterminer le débit de fluide (Qa) dans un accès du sang ayant une position en amont et une position en aval, l'appareil comprenant: une source de liquide de dialyse,

   a. une unité de traitement (11) ayant une membrane semi-perméable (14) délimitant un premier compartiment (12) à travers lequel passe le sang retiré dudit accès du sang, et un deuxième compartiment (13) à travers lequel passe le liquide de dialyse,
   b. une ligne de liquide de dialyse pour faire circuler le liquide de dialyse dans le deuxième compartiment (13) ;
   c. une ligne artérielle (5) reliée à une entrée du premier compartiment (12),
   d. une ligne veineuse (6) reliée à une sortie du premier compartiment (12),
   e. lesdites lignes artérielle et veineuse (5, 6) étant capable d'être configurées selon au moins une configuration normale, dans laquelle ladite ligne artérielle (5) amène le sang de ladite position en amont dudit accès du sang, et ladite ligne veineuse (6) amène le sang vers ladite position en aval dudit accès du sang, et selon au moins une configuration inverse, dans laquelle ladite ligne artérielle (5) amène le sang de ladite position en aval dudit accès du sang, et ladite ligne veineuse (6) amène le sang vers ladite position en amont dudit accès du sang,
   f. moyens pour commuter (28) les lignes veineuse et artérielle (5, 6) pendant un intervalle de temps T entre l'une desdites configurations normale et inverse et l'autre desdites configurations normale et inverse,
   g. moyens pour varier (17, 18) une concentration (Ci) d'au moins une substance du liquide de dialyse en amont de l'unité de traitement,
   h. un capteur (20) opérant en aval de l'unité de traitement (11) pour détecter une conductivité en aval de l'unité de traitement dans le liquide de dialyse ou une concentration en aval de l'unité de traitement de ladite substance dans le liquide de dialyse, et
   i. une unité de commande (85) étant capable d'exécuter les étapes suivantes:

      o actionner lesdits moyens de variation (17, 18) de sorte que, au moins pour ledit intervalle de temps T, ledit liquide de dialyse circulant en amont du deuxième compartiment de l'unité de traitement (11) comprenne au moins une substance ayant une concentration (Ci) différente de la concentration de la même substance dans le sang,
      o obtenir dudit capteur (20) une première conductivité en aval de l'unité de traitement du liquide de dialyse ou une première concentration en aval de l'unité de traitement de ladite substance dans le liquide de dialyse, les lignes veineuse et artérielle (5, 6) étant configurées selon l'une desdites configurations normale et inverse, ladite première conductivité ou concentration se référant au liquide de dialyse avant la commutation des lignes veineuse et artérielle (5, 6) et pendant ledit intervalle de temps T,

o obtenir dudit capteur (20) une deuxième conductivité en aval de l'unité de traitement du liquide de dialyse ou concentration en aval de l'unité de traitement de ladite substance dans le liquide de dialyse, les lignes veineuse et artérielle (5, 6) étant configurées selon l'autre desdites configurations normale et inverse, ladite deuxième conductivité ou concentration se référant au liquide de dialyse après la commutation des lignes veineuse et artérielle et pendant ledit intervalle de temps T,

o calculer le débit de fluide (Qa) dans ledit accès du sang en fonction de la dite première concentration ou conductivité en aval de l'unité de traitement et de ladite deuxième concentration ou conductivité en aval de l'unité de traitement,

**caractérisé en ce que** l'unité de commande (85) est capable d'agir sur les moyens de variation (17, 18) afin de maintenir sensiblement <u>constante</u> la concentration (Ci) de ladite au moins une substance du liquide de dialyse en amont de l'unité de traitement pendant ledit intervalle de temps T.

2. Appareil selon la revendication 1, où ledit capteur comprend une cellule de conductivité en aval de l'unité de traitement (Cn, Cr).

3. Appareil selon la revendication 1, où les moyens de variation sont aptes à augmenter ou réduire la concentration d'une ou plusieurs substances dans le liquide de dialyse.

4. Appareil selon la revendication 1, où pendant ledit intervalle de temps T l'unité de commande (85) agit sur lesdits moyens de commutation pour exécuter les sous-étapes consécutives suivantes:

   a. d'abord configurer lesdites lignes artérielle et veineuse selon la configuration normale pour obtenir ladite première concentration ou première conductivité (Cn), et ensuite
   b. configurer lesdites lignes artérielle et veineuse selon la configuration inverse pour obtenir ladite deuxième concentration ou conductivité (Cr),
   c. ramener les lignes artérielle et veineuse à la configuration normale pour continuer le traitement du sang.

5. Appareil selon la revendication 1, où pendant ledit intervalle de temps T l'unité de commande (85) agit sur lesdits moyens de commutation pour exécuter les sous-étapes consécutives suivantes:

   a. d'abord configurer lesdites lignes artérielle et veineuse selon la configuration inverse pour obtenir ladite première concentration ou conductivité de l'unité de post-traitement (Cr), et ensuite
   b. configurer lesdites lignes artérielle et veineuse selon la configuration normale pour obtenir ladite deuxième concentration ou conductivité (Cn) et ensuite continuer le traitement du sang.

6. Appareil selon la revendication 1, où l'unité de commande (85) est capable d'exécuter une étape pour vérifier si les lignes artérielle et veineuse sont dans ladite configuration normale ou dans ladite configuration inverse.

7. Appareil selon la revendication 4, où après avoir configuré les lignes artérielle et veineuse selon la configuration inverse, l'unité de commande est à même d'exécuter une autre étape pour vérifier si les lignes artérielle et veineuse sont dans ladite première configuration ou dans ladite configuration inverse.

8. Appareil selon la revendication 1, où l'unité de commande (85) est à même d'exécuter les étapes suivantes:

   a. déterminer le débit de transport (Tr) d'ions à travers la membrane semi-perméable,
   b. obtenir la première conductivité en aval de l'unité de traitement (Cn, Cr) se référant au liquide de dialyse avant la commutation des lignes veineuse et artérielle,
   c. obtenir la deuxième conductivité en aval de l'unité de traitement (Cr, Cn) se référant au liquide de dialyse après la commutation des lignes veineuse et artérielle,
   d. calculer le débit de fluide (Qa) dans ledit accès du sang en fonction desdites première et deuxième conductivités de l'unité de post-traitement et dudit débit de transport.

9. Appareil selon la revendication 8, où le débit de fluide (Qa) est calculé à partir des valeurs dudit débit de transport (Tr), de ladite conductivité de l'unité de post-traitement (Cn, Cr), de ladite deuxième conductivité de l'unité de post-traitement (Cr, Cn), et de la conductivité du liquide de dialyse (Ci) en amont de l'unité de traitement.

10. Appareil selon la revendication 8, où lesdites première et deuxième conductivités de l'unité de post-traitement (Cn, Cr) sont obtenues par ledit capteur.

11. Appareil selon une quelconque des revendications 1 à la revendication précédente, comprenant en outre des moyens agissant sur la ligne de dialyse pour causer un débit d'ultrafiltration (Quf).

12. Appareil selon la revendication 9 ou 11, où le débit de fluide (Qa) dans ledit accès du sang est calculé avec la formule Qa = (Tr)*(Cr-Ci)/(Cn-Cr) ou avec la formule Qa = (Tr-Quf)*(Cr-Ci)/(Cn-Cr), où Tr est déterminé à partir des valeurs mesurées in vivo de clearance K ou de dialysance ionique D, obtenues lorsque lesdites lignes veineuse et artérielle sont dans la configuration normale.

13. Appareil selon la revendication 9 ou 11, où $Tr_r$ est le débit de transport obtenu à partir des valeurs mesurées in vivo de clearance K ou de dialysance ionique D lorsque lesdites lignes veineuse et artérielle sont dans la configuration inverse, le débit de fluide (Qa) dans ledit accès du sang étant calculé avec la formule Qa = (Tr-Quf)*(Cr-Ci)/(Cn-Cr) + $Tr_r$.

14. Appareil salon une quelconque des revendications 1 à la revendication précédente, comprenant en outre des moyens pour préparer le liquide de dialyse avec une conductivité différente dudit sang, préférablement ces moyens comprennent des moyens pour mélanger de façon contrôlée les concentrés électrolytique avec l'eau.

15. Appareil selon une quelconque des revendications 1 à la revendication précédente, où lesdits moyens de commutations comprennent:

   - connections à modification manuelle dans la ligne artérielle à la position aval de l'accès du sang, et dans la ligne veineuse à la position amont de l'accès du sang, ou
   - premiers conduits reliant la ligne artérielle aux deux positions en amont et en aval de l'accès du sang, et deuxièmes conduits reliant la ligne veineuse aux deux positions amont et aval de l'accès du sang, des moyens pour fermer de façon sélective l'un des premiers conduits entre la ligne artérielle et l'accès du sang, et des moyens pour fermer de façon sélective l'un des premiers conduits entre la ligne veineuse et l'accès du sang, ou
   - une valve étant capable de relier la ligne artérielle à la position amont du point d'accès et la ligne veineuse à la position aval du point d'accès dans une première position de ladite valve, et étant capable de relier la ligne artérielle à la position aval du point d'accès et la ligne veineuse à la position amont du point d'accès dans une deuxième position de ladite valve, ou
   - moyens de distribution du flux pour relier la ligne artérielle à la position amont du point d'accès et la ligne veineuse à la position aval du point d'accès dans une première condition desdits moyens de distribution du flux, et étant capables de relier la ligne artérielle à la position aval du point d'accès et la ligne veineuse à la position amont du point d'accès dans une deuxième condition desdits moyens de distribution du flux.

16. Appareil selon une quelconque des revendications 1 à la revendication précédente, où ladite unité de traitement (11) comporte l'un des éléments choisis dans le groupe comprenant:

   i. un dialyseur,
   ii. un hémofiltre,
   iii. un plasmafiltre,
   iv. un hémodiafiltre,
   v. un ultrafiltre.

17. Produit logiciel comprenant des instructions à exécuter par une unité de commande (85) d'un appareil de traitement de sang selon une quelconque des revendications 1 à 16, ledit logiciel exécuté dans ladite unité de commande (85) lui permettant la réalisation d'une méthode pour déterminer un débit de fluide (Qa) dans un accès du sang ayant une position amont et une position aval en utilisant ledit appareil de traitement de sang, comprenant les étapes suivantes:

   - faire passer un liquide de dialyse à travers le deuxième compartiment (13) de ladite unité de traitement (11), au moins pour un intervalle de temps T, ledit liquide de dialyse en amont de l'unité de traitement (11) comprenant au moins une substance ayant une concentration (Ci) différente de la concentration de ladite substance dans le sang,

- obtenir en aval du deuxième compartiment (13) de ladite unité de traitement (11) une première conductivité en aval de l'unité de traitement du liquide de dialyse ou une première concentration an aval de l'unité de traitement de ladite substance dans le liquide de dialyse, ladite première conductivité ou concentration (Cn, Cr) se référant aux lignes veineuse et artérielle configurées selon l'une desdites configurations normale et inverse, ladite première conductivité ou concentration en aval de l'unité de traitement se référant au liquide de dialyse avant la commutation des lignes veineuse et artérielle et pendant ledit intervalle de temps T,

- commuter les lignes veineuse et artérielle, pendant ledit intervalle de temps T, entre l'une desdites configurations normale et inverse et l'autre desdites configurations normale et inverse,

- obtenir en aval du deuxième compartiment (13) de ladite unité de traitement (11) une deuxième conductivité en aval de l'unité de traitement du liquide de dialyse ou concentration en aval de l'unité de traitement de ladite substance dans le liquide de dialyse, ladite deuxième conductivité ou concentration (Cn, Cr) se référant aux lignes veineuse et artérielle configurées selon l'autre desdites configurations normale et inverse, ladite deuxième conductivité ou concentration en aval de l'unité de traitement se référant au liquide de dialyse après la commutation des lignes veineuse et artérielle et pendant ledit intervalle de temps T,

calculer ledit débit de fluide (Qa) dans ledit accès du sang en fonction:

- o de ladite première concentration ou conductivité en aval de l'unité de traitement, et
- o de ladite deuxième concentration ou conductivité en aval de l'unité de traitement,

et où pendant ledit intervalle de temps T du passage d'un liquide de dialyse, la concentration Ci de ladite au moins une substance dans le liquide à l'entrée de l'unité de traitement est maintenue sensiblement constante.

**18.** Logiciel selon la revendication 17, ledit logiciel exécuté dans ladite unité de commande (85) lui permettant la réalisation de ladite méthode, où l'étape de faire passer un liquide de dialyse à travers le deuxième compartiment comprend les sous-étapes suivantes:

j. faire passer un premier liquide de dialyse à travers l'entrée du deuxième compartiment de ladite unité de traitement, ledit premier liquide de dialyse ayant une concentration de traitement de ladite substance, ensuite
k, augmenter ou réduire à un point Ti la concentration de la substance dans le liquide de dialyse pour faire passer à travers l'entrée du deuxième compartiment un deuxième liquide qui, pendant l'intervalle de temps T, a une concentration d'au moins ladite substance différente de la concentration de la même substance dans le sang.

**19.** Logiciel selon la revendication 17, ledit logiciel exécuté dans ladite unité de commande (85) lui permettant la réalisation de ladite méthode, où l'étape de faire passer un liquide de dialyse à travers le deuxième compartiment comprend les sous-étapes suivantes:

a. faire passer un premier liquide de dialyse à travers l'entrée du deuxième compartiment de ladite unité de traitement, ledit premier liquide de dialyse ayant une concentration de traitement de substances préétablies, ensuite
b. pendant l'intervalle de temps T, augmenter ou réduire à un point Ti la concentration de plus d'une desdites substances préétablies dans le liquide de dialyse pour faire passer à travers l'entrée du deuxième compartiment un deuxième liquide ayant une concentration desdites substances différente de la concentration des mêmes substances dans le sang.

**20.** Logiciel selon la revendication 18, ledit logiciel exécuté dans ladite unité de commande (85) lui permettant la réalisation de ladite méthode, où ladite substance est un ion.

**21.** Logiciel selon la revendication 19, ledit logiciel exécuté dans ladite unité de commande (85) lui permettant la réalisation de ladite méthode, où lesdites substances sont des ions.

**22.** Logiciel selon la revendication 19, ledit logiciel exécuté dans ladite unité de commande (85) lui permettant la réalisation de ladite méthode, où le débit de fluide (Qa) dans ledit accès du sang est calculé en fonction desdites premières concentrations ou conductivité en aval de l'unité de traitement et desdites deuxièmes concentrations ou conductivité en aval de l'unité de traitement.

23. Logiciel selon la revendication 17, ledit logiciel exécuté dans ladite unité de commande (85) lui permettant la réalisation de ladite méthode, où pendant ledit intervalle de temps T sont prévues les sous-étapes consécutives suivantes:

    a. d'abord configurer lesdites lignes artérielle et veineuse selon la configuration normale pour obtenir ladite première concentration ou première conductivité (Cn), et ensuite
    b. configurer lesdites lignes artérielle et veineuse selon la configuration inverse pour obtenir ladite deuxième concentration ou conductivité (Cr).

24. Logiciel selon la revendication 23, ledit logiciel exécuté dans l'unité de commande (85) lui permettant de réaliser ladite méthode comprenant l'étape de ramener les lignes veineuse et artérielle à la configuration normale pour commencer un traitement de sang.

25. Logiciel selon la revendication 17, ledit logiciel exécuté dans ladite unité de commande (85) lui permettant la réalisation de ladite méthode, où pendant ledit intervalle de temps T sont prévues les sous-étapes consécutives suivantes:

    a d'abord configurer lesdites lignes artérielle et veineuse selon la configuration inverse pour obtenir ladite première concentration ou conductivité en aval de l'unité de traitement (Cr), et ensuite
    b. configurer lesdites lignes artérielle et veineuse selon la configuration normale pour obtenir ladite deuxième concentration ou conductivité (Cn).

26. Logiciel selon la revendication 17, ledit logiciel exécuté dans ladite unité de commande (85) lui permettant la réalisation de ladite méthode, où il est prévu une étape pour contrôler si les lignes artérielle et veineuse sont dans ladite configuration normale ou dans ladite configuration inverse.

27. Logiciel selon la revendication 23, ledit logiciel exécuté dans ladite unité de commande (85) lui permettant la réalisation de ladite méthode, où après avoir configuré les lignes artérielle et veineuse selon la configuration inverse, il est prévu une autre étape pour contrôler si les lignes artérielle et veineuse sont dans ladite configuration normale ou dans ladite configuration inverse.

28. Logiciel selon la revendication 26 ou 27, ledit logiciel exécuté dans ladite unité de commande (85) lui permettant la réalisation de ladite méthode, où l'étape pour contrôler si les lignes artérielle et veineuse sont dans la configuration normale ou inverse comporte les étapes suivantes:

    a. déterminer la valeur in vivo d'un paramètre choisi dans le groupe comprenant:

        i. dialysance ionique réelle D ou
        ii. clearance réelle K ou
        iii. un paramètre proportionnel à la dialysance ionique réelle ou
        iv. un paramètre proportionnel à la clearance réelle,

    b. comparer la valeur in vivo dudit paramètre avec une valeur de seuil correspondante si les lignes veineuse et artérielle sont dans ladite configuration normale ou dans ladite configuration inverse.

29. Logiciel selon la revendication 28, ledit logiciel exécuté dans ladite unité de commande (85) lui permettant la réalisation de ladite méthode, où l'étape de déterminer la valeur in vivo dudit paramètre comporte les étapes suivantes:

    a. faire passer un troisième liquide de dialyse à travers l'entrée du deuxième compartiment de ladite unité de traitement, ledit liquide de dialyse ayant une concentration d'au moins une substance, ensuite
    b. obtenir une troisième conductivité en aval de l'unité de traitement du liquide de dialyse ou troisième concentration en aval de l'unité de traitement de ladite substance dans le troisième liquide de dialyse,
    c. au moins pour un deuxième intervalle de temps, augmenter ou réduire la concentration de la substance dans le troisième liquide de dialyse pour faire passer un quatrième liquide à travers l'entrée du deuxième compartiment, ledit quatrième liquide ayant une concentration d'au moins ladite substance différente de la concentration de la même substance dans le troisième liquide,
    d. obtenir une quatrième conductivité en aval de l'unité de traitement du liquide de dialyse ou quatrième concentration en aval de l'unité de traitement de ladite substance dans le quatrième liquide de dialyse,
    e. calculer la valeur in vivo dudit paramètre en fonction de ladite troisième concentration ou conductivité en aval de l'unité de traitement et de ladite quatrième concentration ou conductivité en aval de l'unité de traitement.

**30.** Logiciel selon la revendication 28, ledit logiciel exécuté dans ladite unité de commande (85) lui permettant la réalisation de ladite méthode, où ladite valeur de seuil est une valeur préétablie ou une valeur calculée ou une valeur mesurée.

**31.** Logiciel selon la revendication 28, ledit logiciel exécuté dans ladite unité de commande (85) lui permettant la réalisation de ladite méthode, où l'étape de détermination in vivo dudit paramètre est exécutée pendant l'intervalle de temps T.

**32.** Logiciel selon la revendication 28, ledit logiciel exécuté dans ladite unité de commande (85) lui permettant la réalisation de ladite méthode, où il est prévu une autre étape pour envoyer un signal d'alarme au cas où l'étape de comparaison établit que les lignes veineuse et artérielle sont dans ladite configuration inverse.

**33.** Logiciel selon la revendication 26, ledit logiciel exécuté dans ladite unité de commande (85) lui permettant la réalisation de ladite méthode, où l'étape pour contrôler si les lignes artérielle et veineuse sont dans ladite configuration normale ou dans ladite configuration inverse est exécutée pendant ledit premier intervalle de temps T.

**34.** Logiciel selon la revendication 17 et selon la revendication 33, ledit logiciel exécuté dans ladite unité de commande (85) lui permettant la réalisation de ladite méthode, où l'étape pour contrôler si les lignes artérielle et veineuse sont dans ladite configuration normale ou dans ladite configuration inverse comprend les sous-étapes suivantes:

- comparer ladite première conductivité en aval de l'unité de traitement du liquide de dialyse ou première concentration en aval de l'unité de traitement de ladite substance dans le liquide de dialyse ainsi obtenue avec ladite deuxième conductivité en aval de l'unité de traitement du liquide de dialyse ou deuxième concentration en aval de l'unité de traitement de ladite substance dans le liquide de dialyse ainsi obtenue,
- déterminer si ladite conductivité ou concentration augmentent après l'étape de commutation.

**35.** Logiciel selon la revendication 17, ledit logiciel exécuté dans ladite unité de commande (85) lui permettant la réalisation de ladite méthode, comprenant en outre les étapes suivantes:

a. déterminer le débit de transport (Tr) d'ions à travers la membrane semi-perméable,
b. obtenir la première conductivité en aval de l'unité de traitement (Cn; Cr) pour le liquide de dialyse avant la commutation des lignes veineuse et artérielle,
c. obtenir la deuxième conductivité en aval de l'unité de traitement (Cr; Cn) pour le liquide de dialyse après la commutation des lignes veineuse et artérielle.
d. calculer le débit de fluide (Qa) dans ledit accès du sang en fonction desdites première et deuxième conductivités en aval de l'unité de traitement et dudit débit de transport.

**36.** Logiciel selon la revendication 18, ledit logiciel exécuté dans ladite unité de commande (85) lui permettant la réalisation de ladite méthode, où le débit de fluide (Qa) est calculé à partir des valeurs dudit débit de transport (Tr), de ladite première conductivité en aval de l'unité de traitement (Cn), de ladite deuxième conductivité en aval de l'unité de traitement (Cr), et de la conductivité du liquide de dialyse (Ci) en amont de l'unité de traitement.

**37.** Logiciel selon la revendication 35 ou 36, ledit logiciel exécuté dans ladite unité de commande (85) lui permettant la réalisation de ladite méthode, où lesdites première et deuxième conductivités en aval de l'unité de traitement (Cn, Cr) sont obtenues en mesurant la conductivité du fluide effluent en sortie du deuxième compartiment de ladite unité de traitement.

**38.** Logiciel selon une quelconque des revendications précédentes, ledit logiciel exécuté dans ladite unité de commande (85) lui permettant la réalisation de ladite méthode, comprenant en outre l'étape d'obtenir le débit d'ultrafiltration (Quf).

**39.** Logiciel selon la revendication 36, ledit logiciel exécuté dans ladite unité de commande (85) lui permettant la réalisation de ladite méthode, où le débit de fluide (Qa) dans ledit accès du sang est calculé avec la formule $Qa = (Tr)*(Cr-Ci)/(Cn-Cr)$.

**40.** Logiciel selon la revendication 38, ledit logiciel exécuté dans ladite unité de commande (85) lui permettant la réalisation de ladite méthode, où le débit de fluide (Qa) dans ledit accès du sang est calculé avec la formule $Qa = (Tr-Quf)*(Cr-Ci)/(Cn-Cr)$.

**41.** Logiciel selon la revendication 38, ledit logiciel exécuté dans ladite unité de commande (85) lui permettant la réalisation de ladite méthode, où Tr est déterminé à partir des valeurs mesurées in vivo de clearance K ou de dialysance ionique D, obtenues lorsque lesdites lignes veineuse et artérielle sont dans la configuration normale, le débit de fluide (Qa) dans ledit accès du sang étant calculé ou avec la formule Qa = (Tr-Quf)*(Cr-Ci)/(Cn-Cr).

**42.** Logiciel selon la revendication 38, ledit logiciel exécuté dans ladite unité de commande (85) lui permettant la réalisation de ladite méthode, où Tr, est le débit de transport d'ions à travers la membrane semi-perméable déterminé à partir des valeurs mesurées in vivo de clearance K ou de dialysance ionique D, obtenues lorsque lesdites lignes veineuse et artérielle sont dans la configuration inverse, le débit de fluide (Qa) dans ledit accès du sang étant calculé avec la formule Qa = (Tr-Ouf)*(Cr-Ci)/(Cn-Cr) + $Tr_r$.

**43.** Logiciel selon la revendication 41 ou la revendication 42, ledit logiciel exécuté dans ladite unité de commande (85) lui permettant la réalisation de ladite méthode, où les valeurs mesurées in vivo de clearance K ou de dialysance ionique D sont obtenues par les étapes suivantes:

a. faire passer un troisième liquide de dialyse à travers le deuxième compartiment (13) de ladite unité de traitement, ledit liquide de dialyse ayant une concentration d'au moins une substance, ensuite
b. obtenir une troisième conductivité en aval de l'unité de traitement du liquide de dialyse ou troisième concentration en aval de l'unité de traitement de ladite substance dans le troisième liquide de dialyse,
c. au moins pour un deuxième intervalle de temps, augmenter ou réduire la concentration de la substance dans le troisième liquide de dialyse pour faire passer un quatrième liquide à travers l'entrée du deuxième compartiment, ledit quatrième liquide ayant une concentration d'au moins ladite substance différente de la concentration de la même substance dans le troisième liquide,
d. obtenir une quatrième conductivité en aval de l'unité de traitement du liquide de dialyse ou quatrième concentration en aval de l'unité de traitement de ladite substance dans le quatrième liquide de dialyse,
e. calculer la valeur in vivo de K ou D en fonction de ladite troisième concentration ou conductivité en aval de l'unité de traitement et de ladite quatrième concentration ou conductivité en aval de l'unité de traitement.

**44.** Logiciel selon la revendication 35 ou 40, ledit logiciel exécuté dans ladite unité de commande (85) lui permettant la réalisation de ladite méthode, où le débit de transport (Tr) correspond à:

- valeurs connues d'un certain dialyseur, ou
- valeurs calculées, ou
- dialysance ionique réelle mesurée (D), ou
- en utilisant un échantillon de sang pré-dialyse avec la concentration d'urée initiale dans le liquide de dialyse, ou
- clearance K mesurée du dialyseur, préférablement la valeur de clearance de l'urée.

**45.** Logiciel selon la revendication 41 ou 42, ledit logiciel exécuté dans ladite unité de commande (85) lui permettant la réalisation de ladite méthode, où la clearance mesurée K ou la dialysance ionique mesuré D sont des valeurs in vivo déterminées pendant l'intervalle de temps T.

**46.** Logiciel selon la revendication 18, ledit logiciel exécuté dans ladite unité de commande (85) lui permettant la réalisation de ladite méthode, où le troisième liquide correspond au premier liquide et le quatrième liquide correspond au deuxième liquide.

**47.** Logiciel selon la revendication 45 et la revendication 21, ledit logiciel exécuté dans ladite unité de commande (85) lui permettant la réalisation de ladite méthode, où le troisième liquide correspond au premier liquide et le quatrième liquide correspond au deuxième liquide.

**48.** Logiciel selon la revendication 18 ou 19, ledit logiciel exécuté dans ladite unité de commande (85) lui permettant la réalisation de ladite méthode, où pendant ledit intervalle de temps T la conductivité du deuxième liquide est plus élevée que celle du premier liquide.

**49.** Logiciel selon la revendication 48, ledit logiciel exécuté dans ladite unité de commande (85) lui permettant la réalisation de ladite méthode, où pendant ledit intervalle de temps T la conductivité du deuxième liquide est au moins 1 mS/cm (1 milli-Siemens/centimètre) plus élevée que celle du premier liquide.

**50.** Logiciel selon la revendication 49, ledit logiciel exécuté dans ladite unité de commande (85) lui permettant la réalisation de ladite méthode, où pendant ledit intervalle de temps T la conductivité du deuxième liquide est au moins 2 mS/cm (2 milli-Siemens/centimètre) plus élevée que celle du premier liquide, si la conductivité du premier liquide est moins que ou égale à 15 mS/cm.

51. Logiciel selon une quelconque des revendications 48, 49, 50. ledit logiciel exécuté dans ladite unité de commande (85) lui permettant la réalisation de ladite méthode, comprenant les étapes suivantes:

> a. varier la conductivité du premier liquide en amont de l'unité de traitement (11) pour définir le deuxième liquide,
> b. maintenir sensiblement constante pendant ledit intervalle de temps T la conductivité du deuxième liquide en amont de l'unité de traitement (11),
> c. attendre un certain temps après le début de la variation de conductivité et ensuite déterminer le temps T0 auquel se passe une variation préétablie de conductivité dans le liquide en aval du dialyseur (11),
> d. mesurer une pluralité de premières valeurs de la conductivité du liquide en aval de l'unité de traitement (11) après ledit temps T0,
> e. calculer la première conductivité en aval de l'unité de traitement dudit liquide à partir de ladite pluralité de valeurs,
> f. commuter les lignes de l'une desdites configurations à l'autre desdites configurations,
> g. mesurer une pluralité de deuxièmes valeurs de la conductivité du liquide en aval de l'unité de traitement (11) après ladite commutation de temps,
> h. calculer la deuxième conductivité en aval de l'unité de traitement dudit liquide à partir de ladite pluralité de valeurs.

52. Logiciel selon la revendication 51, ledit logiciel exécuté dans ladite unité de commande (85) lui permettant la réalisation de ladite méthode, où il est prévu une autre étape pour varier la conductivité dans le deuxième liquide en amont de l'unité de traitement (11).

53. Logiciel selon la revendication 51, ledit logiciel exécuté dans ladite unité de commande (85) lui permettant la réalisation de ladite méthode, où le mesurage des premières valeurs est exécuté après un certain retard du temps T0.

54. Logiciel selon la revendication 53, ledit logiciel exécuté dans ladite unité de commande (85) lui permettant la réalisation de ladite méthode, où on détermine l'instant $T_{rev}$ auquel se passe la commutation, le mesurage des deuxièmes valeurs étant exécuté après un retard du temps $T_{rev}$.

55. Logiciel selon la revendication 54, ledit logiciel exécuté dans ladite unité de commande (85) lui permettant la réalisation de ladite méthode, où la pluralité de deuxièmes valeurs de concentration ou conductivité après l'étape de commutation est mesurée de façon continue ou intermittente, et la concentration ou conductivité (Cr) à l'instant de la commutation $T_{rev}$ est déterminée par extrapolation des valeurs mesurées en arrière à l'instant ($T_{rev}$) de la commutation.

56. Logiciel selon une quelconque des revendications 17 à la revendication précédente, ledit logiciel exécuté dans ladite unité de commande (85) lui permettant la réalisation de ladite méthode, où la conductivité du fluide de dialyse est réglée en variant la concentration en chlorure de sodium, ou en variant la concentration en tous les électrolytes A-concentrés, ou en variant la concentration en tous les électrolyte dans le liquide de dialyse.

57. Produit logiciel selon une quelconque des revendications 17 à 56, où il est enregistré dans un support de données magnétique ou optique.

58. Produit logiciel selon une quelconque des revendications 17 à 56, où il est enregistré dans la mémoire d'un ordinateur.

59. Produit logiciel selon une quelconque des revendications 17 à 56, ou 57, ou 58, où il est enregistré dans une mémoire morte.

60. Produit logiciel selon une quelconque des revendications 17 à 56, où il est enregistré dans un ordinateur à distance de l'appareil de traitement de sang et il est capable d'être transmis sur un signal électrique ou électromagnétique.

Fig.1

Fig.2

Fig. 3

Fig. 4

FIG 5

**FIG 6**

**EP 1 471 957 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5685989 A
- US 5595182 A
- US 5453576 A
- US 5510716 A
- US 5510717 A
- US 5392550 A
- EP 928614 A
- WO 0024440 A
- EP 0928614 A
- US 5605630 A
- US 5894011 A
- EP 658352 A